(19) Europäisches Patentamt
European Patent Office
Office européen des brevets

(11) EP 1 698 638 A1

(12) **EUROPEAN PATENT APPLICATION**

(43) Date of publication:
**06.09.2006 Bulletin 2006/36**

(21) Application number: **05004582.2**

(22) Date of filing: **02.03.2005**

(51) Int Cl.:
*C07K 9/00* (2006.01) *A61K 38/14* (2006.01)
*C12P 21/00* (2006.01) *C12R 1/01* (2006.01)
*A61P 31/00* (2006.01) *A61P 35/00* (2006.01)

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(83) **Declaration under Rule 28(4) EPC (expert solution)**

(71) Applicant: **Technische Universität Berlin**
**10623 Berlin (DE)**

(72) Inventors:
- **Neuhof, Torsten**
  **14197 Berlin (DE)**
- **Dieckmann, Ralf**
  **10707 Berlin (DE)**
- **von Döhren, Hans**
  **10627 Berlin (DE)**
- **Preussel, Karina**
  **12524 Berlin (DE)**
- **Seibold, Michael**
  **13509 Berlin (DE)**
- **Schmieder, Peter**
  **16540 Hohen Neuendorf (DE)**

(74) Representative: **Engelhard, Markus**
**Forrester & Boehmert**
**Pettenkoferstrasse 20-22**
**80336 München (DE)**

(54) **Lipopeptides having pharmaceutical activity**

(57) The present invention relates to lipopeptides having pharmaceutical activity, to methods of their isolation and production, to pharmaceutical composition and uses thereof and to cyanobacteria from which the compounds may be isolated.

EP 1 698 638 A1

## Description

The present invention relates to lipopeptides having pharmaceutical activity, to methods of their isolation and production, to pharmaceutical composition and uses thereof and to cyanobacteria from which the compounds may be isolated.

Natural products have paramount importance in the development of new pharmaceutical products. 15 out of 35 of the most important "block-buster-drugs" of the years 2000 - 2003 are directly derived from natural products or represent a modification of such natural products (Butler, M., the Role of Natural Product Chemistry in Drug Discovery, *J. Nat. Prod.* 2004, 67, 2141-2153). Natural products occur in all fields of pharmaceutical drug production, such as anti-cancer agents, anti-infectious agents, immuno-suppressants and other therapeutics. Natural products of diverse structures are produced by microorganisms in many cases, wherein peptide antibiotics represents a large subclass of the known antibacterial and antifungal agents, that can be isolated from various prokaryotic and eukaryotic microorganisms. Peptide antibiotics and structurally similar agents have a number of interesting biological effects. For example, some peptide antibiotics are known to be effective antifungal agents. Data from the statistics of the "National Center for Health" in the US show that the number of invasive fungal infections has continuously increased over the last years. In 1980 828 mortalities in US hospitals caused by fungal infections were registered. In 1997, the number of mortalities increased to 2370 *(Arzneimitteltherapie* 22. Jahrgang Heft 12 2004). The causative agent for invasive fungal infections are *Candida* species and *Aspergillus* species which are responsible for the mycoses commonly encountered in particular in those patients with an impaired immune system for example after having received an organ transplantation, during chemotherapy, or those patients having AIDS.

Presently available antifungal agents have a limited therapeutic application because of considerable side effects, development of resistance, suboptimal pharmacogenetic qualities and high costs during long-term therapies. Presently available antifungal agents can be essentially divided into eight different classes: Allylamines, benzofuranes, imidazoles, morpholines, polyenes, pyridones, pyrimidines and triazols. Their antifungal effect is based on their activity on o ne o r several of the following: cytoplasmic membrane, nucleic a cid synthesis, nuclear division, enzyme regulation and respiratory chain of the fungal organism.

A new c lass of antifungal agents are the echinocandins and their derivatives. A prominent member of this class is the semi-synthetic caspofungin, which is isolated from a fermentation product of the fungus *Glarea lozoyensis* (see Valerie Ledger-Brue et al., 2003, *Journal of Antimicrobial Chemotherapy,* 51, 513-521). It is a derivative of pneumocandin $B_0$. Caspofungin is believed to exert its antifungal activity by inhibiting the synthesis of the fungal cell wall. It blocks the enzyme β(1,3)-D-Glucan-synthase, β(1,3)-D-glucan being an essential component of the fungal cell wall. However, the echinocandins are known to have likewise side effects. Furthermore, t hey have a 1 imited b io-availability which makes t heir u se as pharmaceutical agents less attractive. Also, echinocandins are known to have no or very little activity against *Fusarium* sp., *Cryptococcus neoformans* and *Pseudallescheria sp.* i.e. they have only a limited spectrum of fungi against which they are active. Also, the echinocandins have been difficult to produce, in that they need to be isolated from fungi themselves which may be difficult to cultivate.

Accordingly, it was an object of the present invention to provide for novel antifungal agents that can be produced easily. Furthermore, it was an object of the present invention to provide for antifungal agents that have an enhanced bio-availability and that can for example be applied orally. It was also an object of the present invention to provide for antifungal agents that have a wider spectrum and are, for example, active against *Fusarium* sp. and *Cryptococcus neoformans.* Moreover, it was an object of the present invention to provide for an antifungal agent that allows the production in large quantities.

All these objects are solved by a compound of formula

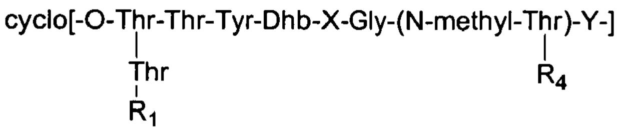

,

wherein

Dhb = 2-amino-2-dehydrobutyric acid

X = glutamine, glutamic acid, or ornithine

Y = glutamine, glutamic acid, or ornithine, or

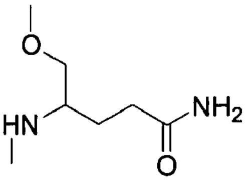

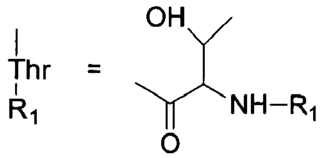

,

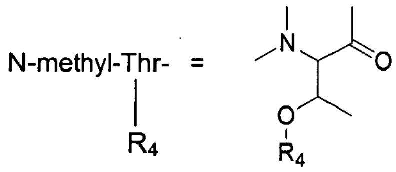

,

$R_1$ =

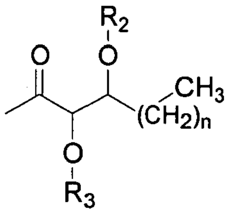

,

or H, wherein n = 0-12,
$R_2$ = hexose, hexulose, pentose, sialic acid or H, in particular rhamnose,
$R_3$ = hexose, hexulose, pentose, sialic acid or H, in particular rhamnose,
$R_4$ = hexose, hexulose, pentose, sialic acid or H, in particular mannose.

This compound may alternatively also be represented by formula

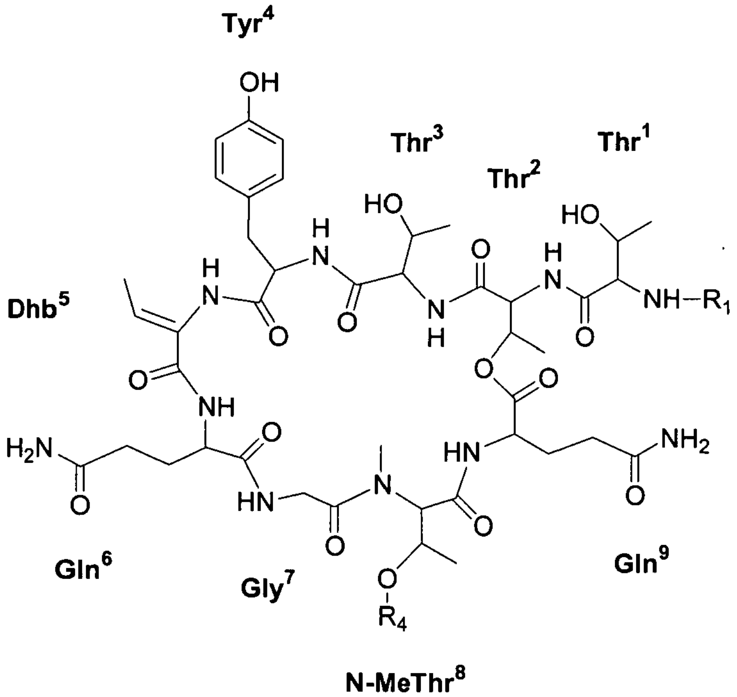

wherein Dhb is as defined before, $Gln^6$ may be glutamine (as shown) or glutamic acid or ornithine, and $Gln^9$ may be glutamine, glutamic acid, ornithine or

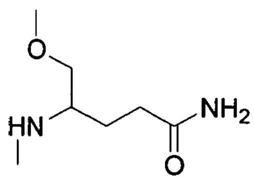

In one embodiment each amino acid is in its D- or L -form, or, in the case of Dhb, in its E- or Z-form.

Preferably, one or several of the OH-groups and/or $NH_2$-groups may be one or several of the following: methylated, phosphorylated, glycosylated, sulfonated, acetylated and, in the case of OH-groups, modified with a $-(CH_2)_2-NH_2$-residue, and/or wherein one or several of the NH-groups may be methylated.

In one embodiment, 2-amino-2-dehydrobutyric acid (Dhb) has been modified such that it has the formula:

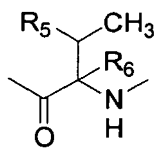

$R_5$ = H, Cl, Br, OH

$R_6$ = H, Cl, Br, OH.

In one embodiment, one of the threonine residues is D-threonine, one is L-threonine, and one is D-allo-threonine, wherein X, Y and N-methyl-Thr are in their D- or L-forms, and wherein Tyr is in its D-form.

In one embodiment, $R_4$ is mannose, $R_1$ is

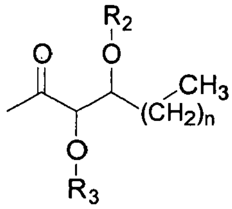

,

wherein n = 10,
$R_2$ = rhamnose or H,
and $R_3$ = H.

Preferably, X is Gln and Y is Gln.

The objects of the present invention are also solved by a compound having the formula

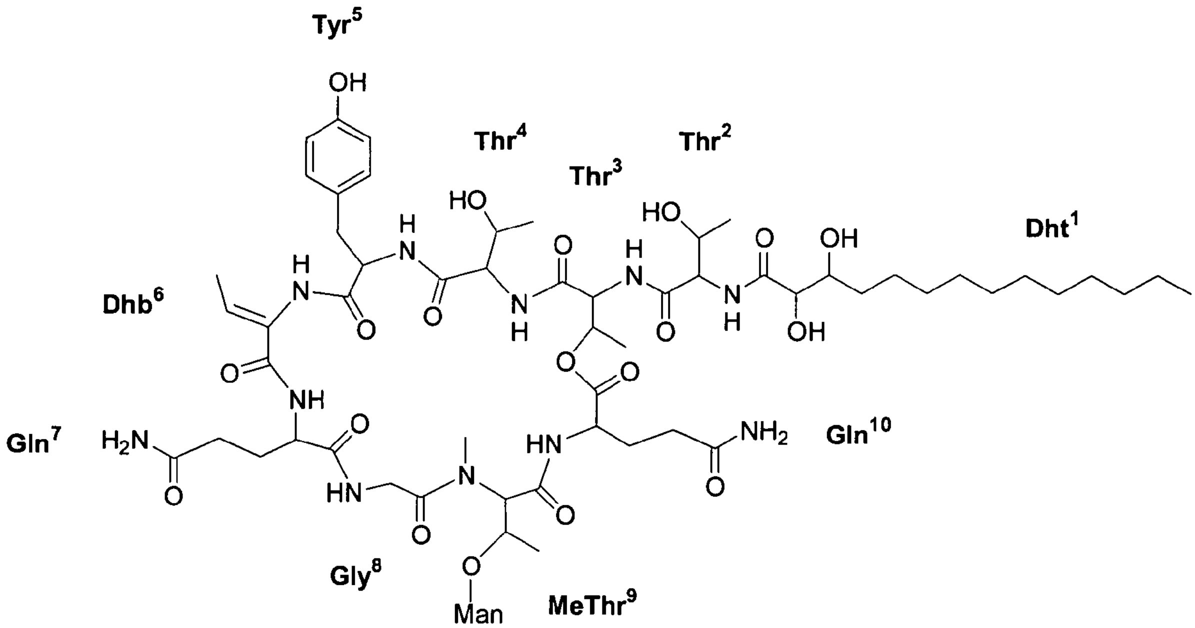

wherein "Dhb" means "2-amino-2-dehydrobutyric acid" "Man" means mannose, "MeThr" means "N-methyl-threonine", and "Dht" means "α,β-dihydroxytetradecanoic acid".

This compound is also sometimes herein referred to as "hassallidin A".

The objects of the present invention are also solved by a compound having the formula

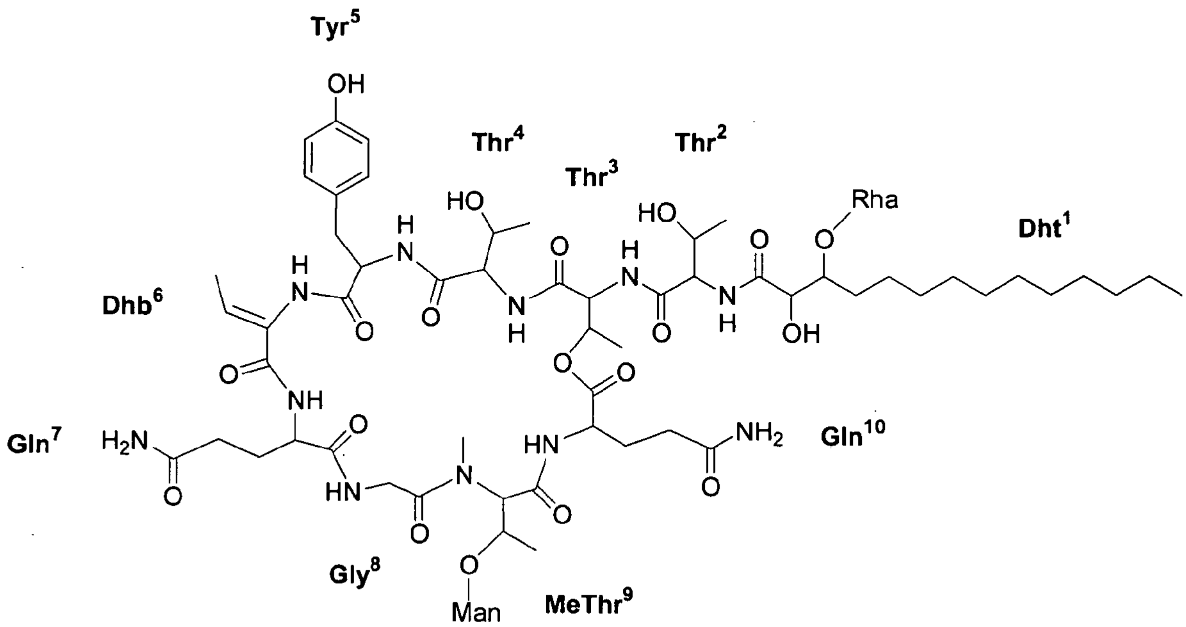

wherein "Dhb" means "2-amino-2-dehydrobutyric acid" "Man" means mannose "Rha" means rhamnose, "MeThr" means "N-methyl-threonine", and "Dht" means "α,β-dihydroxytetradecanoic acid".

This compound is herein also sometimes referred to as "hassallidin B".

It should be clear that the formulae of hassallidin A and B and the more general formula given on page 4 are clear also without the various three- and five-letter abbreviations attached to the various residues, which abbreviations merely serve the purpose of further illustrating the types of residues appearing at each occurrence.

The objects of the present invention are also solved by a pharmaceutical composition comprising a compound according to the present invention, which composition, preferably additionally comprises a pharmaceutically acceptable carrier.

The objects of the present invention are furthermore solved by the use of a compound according to the present invention for the manufacture of a medicament for the treatment of a fungal disease.

The objects of the present invention are furthermore solved by the use of a compound according to the present invention for the manufacture of a medicament for the treatment of a cancerous disease.

The objects of the present invention are also solved by a method of isolating a compound according to the present invention, comprising the following steps:

- growing cyanobacterium HAS BO7, having the official DSMZ accession no. DSM 17156, or *Tolypothrix* sp. in culture,
- extracting the cultivated cyanobacteria with a solvent, preferably an alcoholic solvent, more preferably methanol or ethanol,
- subjecting the resulting extract to a reverse-phase chromatography on HPLC, more preferably eluting with an increasing amount of a non-polar solvent.
  Preferably, the method of isolating such compound further comprises the additional step:
    - identifying the compound by UV-detection at 220 nm.

The objects of the present invention are also solved by a cyanobacterium, as deposited with the DSMZ (Braunschweig, Germany, Deutsche Sammlung für Mikroorganismen und Zellkulturen), under reference sign HAS BO7, and having the official DSMZ accession no. DSM 17156.

As used herein, the term hexose is meant to encompass all aldose carbohydrates having 6 carbon atoms, such as glucose, galactose, mannose, rhamnose, N-acetylglucosamine, N-acetylgalactosamine. The term "hexulose", as used herein is meant to refer to all ketose carbohydrates having 6 carbon atoms, such as fructose, sorbose, psicose. The term "pentose" is meant to refer to carbohydrates having 5 carbon atoms, in particular for example arabinose, ribose, xylose and lyxose. The term "sialic acid", as used herein, is meant to refer to acylneuraminic acids. An example is N-acetyl neuraminic acid.

Unless otherwise specified, the three-letter-code for amino acids is used herein (as e.g. explained in Stryer, "Biochemistry", $3^{rd}$ ed., 1988, page 21, table 2-2). In particular, the abbreviations Thr, Tyr, Gln, and Gly are meant to designate the amino acids threonine, tyrosine, glutamine and glycine. The term N-methyl-Thr (or MeThr) is meant to designate the amino acid N-methyl-threonine. However, it should be noted that Gln, at places specifically indicated, may also mean glutamic acid, ornithine or a glutamine wherein the main chain carbonyl-group has been reduced to $-CH_2$, i.e.

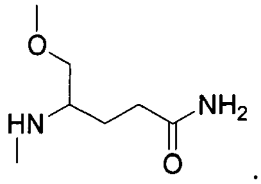

The abbreviations "Man" and "Rha" designate the carbohydrates "mannose" and "rhamnose", respectively.
"Dht", as used herein, is meant to designate α,β-dihydroxytetradecanoic acid. As outlined above, residues "X" and "Y" in the general formula of claim 1, but also in the positions 6 ($Gln^6$) and 9 ($Gln^9$) of the more specific formula thereafter (or in the positions 7 and 10 in the formulae showing hassallidin A and B) can be independently either glutamine, glutamic acid or ornithine. Someone skilled in the art of organic synthesis knows how to convert one amino acid into the other by appropriate amidation (Glu→Gln) and/or reduction (Glu→ornithine). Moreover Y may be

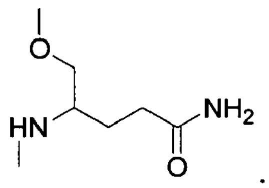

The term "glycosylated", as used herein, is meant to designate the formation of a glycosidic bond between a carbohydrate and a second molecule, for example a peptide. The carbohydrate may be a sugar but also an acid, such as N-acyl neuraminic acids etc. (sialic acids).
As outlined before, also the 2-amino-2-dehydrobutyric acid (Dhb) may have been modified such that it has residues added to its double bond, such that, in effect, the double bond vanishes and becomes fully saturated. Furthermore, it is also possible that the lactone of the cyclic peptide of the compounds according to the present invention is reduced from a lactone to an ether, wherein, in effect, the carbonyl group of residue no. 9, (which may be glutamine, glutamate or ornithine) has been reduced, thus effectively making an ether.
It should also be clear that a pharmaceutical composition according to the present invention may, in addition to the compound according to the present invention, also comprise other pharmaceutical ingredients, such as other antifungal agents, but also for example antiinflammatory a gents. Moreover, a pharmaceutical composition according to the present invention may comprise more than just one type of compound according to the present invention. More specifically, in such pharmaceutical compositions, the compound(s) according to the present invention may be combined with polyenes and/or azoles. For example, they may be combined with amphotericin B and/or clotrimazole. Other combinations are possible with allylamines, benzofuranes, imidazoles, morpholines, pyridones, pyrimidines and triazols.
The present inventors have isolated a new class of lipopeptides, preferably glycosylated lipopeptides, which, surprisingly, show pharmaceutical activity. In particular, the compounds according to the present invention have a broad antifungal activity and an activity against cancerous cells.
Although the inventors have isolated the compounds according to the present invention from a naturally occurring cyanobacterium which has also been deposited with the DSMZ, Braunschweig, Germany under the reference sign HAS BO7 on February 22, 2005, the compounds according to the present invention can also be synthesized easily by someone skilled in the art knowing the structural formula provided by the present inventors. Such syntheses of cyclic lipopeptides and glycosilated cyclic lipopeptides have for example been described in A. Gu W, Silverman RB.. *J Org Chem.* 2003 Nov 14; 68(23):8774-9; Rew Y, Shin D, Hwang I, Boger DL, *J Am Chem Soc.* 2004 Feb 4; 126(4):1041-3; Klein LL, Li L, Chen HJ, Curty CB, DeGoey DA, Grampovnik DJ, Leone CL, Thomas SA, Yeung CM, Funk KW, Kishore V, Lundell EO, Wodka D, Meulbroek JA, Alder JD, Nilius AM, Lartey PA, Plattner JJ, *Bioorg Med Chem.* 2000 Jul; 8(7): 1677-96; and Chen J, Forsyth CJ, Proc *Natl Acad Sci USA.* 2004 Aug 17;101(33):12067-72.

Furthermore, if there are possible stereoisomers, the various possible configurations can be synthesized using combinatorial chemistry as commonly practiced in many organic synthesis labs and as for example described in Moitessier N, Dufour S, Chretien F, Thiery JP, Maigret B, Chapleur Y, Design, synthesis and preliminary biological evaluation of a focused combinatorial library of stereodiverse carbohydrate-scaffold-based peptidomimetics, *Bioorg Med Chem.* 2001 Feb;9(2):511-23;
Okamoto N, Hara O, Makino K, Hamada Y, Diastereoselective synthesis of all stereoisomers of beta-methoxytyrosine, a component of papuamides, *J Org Chem.* 2002 Dec 27;67(26):9210-5;
Moerman MC, Anteunis MJ, Partial synthesis of five new analogues of the peptido-lactone Virginiamycine S1, modified in the fifth and/or sixth position ([Xxx5]-VS1 with Xxx = Ala, Asp, Asn and Lys and [Ala5,Gly6]-VS1), *Int JPept Protein Res.* 1993 Feb;41(2):102-17; and
Yanai M, Hiramoto S, First total synthesis of N-4909 and its diastereomer; a stimulant of apolipoprotein E secretion in human hepatoma Hep G2 cells, *J Antibiot* (Tokyo). 1999 Feb;52(2):150-9. Hence, by providing the formula of the compounds according to the present invention, someone skilled in the art has sufficient information to produce these compounds based on his knowledge of the art of organic synthesis.

In general, peptide sequences can be easily synthesized by standard solid-phase Fmoc chemistry and the crude peptides are cyclized using dimethylsulfoxide, as e.g. described in McBride, J.D., Freeman, N., Domingo, G.J. & Leatherbarrow, R.J. (1996) Selection of chymotrypsin inhibitors from a conformationally-constrained combinatorial peptide library. *J. Mol. Biol.* 259, 819-827.
Series of derivatives of cyclic peptides in which e.g. amino acid residues are replaced can be rapidly synthesized and evaluated, as e.g. described in Zambias, R.A., Hammond, M.L., Heck, J. V., Bartizal, K., Trainor, C., A bruzzo, G., S chmatz, D.M., N ollstadt, K.M. (1992) Preparation and structure-activity relationships of simplified analogues of the antifungal agent ciclofungin: a total synthesis approach. *J.Med.Chem.* 35:2843-2855.
The rapid synthesis and evaluation of large pools of peptide derivatives is possible using methods of solid-phase synthesis and combinatorial chemistry. Synthetic (cyclic) peptide combinatorial libraries (SPCLs), each composed of tens of millions of peptides, can be used for the rapid screening of bioactive peptides, as e.g. described in Pinilla, C., Appel, J.R., Houghten, R.A. (1993) Synthetic peptide combinatorial libraries (SPCLs): identification of antigenic determinant of beta-endorphin recognized by monoclonal antibody 3E7. *Gene,* 128:71-76.

Both side-chain modification (glycosylation and acylation) of the cyclic peptide backbone can be performed chemically and/or enzymatically, as e.g. described in Debono M, Abbott BJ, Molloy RM, Fukuda DS, Hunt AH, Daupert VM, Counter FT, Ott JL, Carrell CB, Howard LC, et al, Enzymatic and chemical modifications of lipopeptide antibiotic A21978C: the synthesis and evaluation of daptomycin (LY146032), *JAntibiot* (Tokyo). 1988 Aug;41(8):1093-105;
Debono M, Abbott BJ, Fukuda DS, Barnhart M, Willard KE, Molloy RM, Michel KH, Turner JR, Butler TF, Hunt AH, Synthesis of new analogs of echinocandin B by enzymatic deacyla-tion and chemical reacylation of the echinocandin B peptide: synthesis of the antifungal agent cilofungin (LY121019), *JAntibiot* (Tokyo). 1989 Mar;42(3):389-97;
Nakahara Y, Iijima H, Shibayama S, Ogawa T, Stereoselective total synthesis of glycopeptides bearing the dimeric and trimeric sialosyl-Tn epitope, *Carbohydr Res.* 1991 Sep 2;216:211-25;
Furstner A, Jeanjean F, Razon P, Wirtz C, Mynott R, Total synthesis of woodrosin I--part 1: preparation of the building b locks and evaluation of the glycosylation strategy, *Chemistry.* 2003 Jan 3;9(1):307-19; and
Yuan Y, Men H, Lee C, Total synthesis of kendomycin: a macro-C-glycosidation approach, J *Am Chem Soc.* 2004 Nov 17;126(45):14720-1.
Lane JW, Halcomb RL, Stereoselective synthesis of conformationally constrained g lycosy-lated amino acids using an enzyme-catalyzed desymmetrization, *J Org Chem.* 2003 Feb 21;68(4):1348-57.

The present inventors have identified lipopeptides which have an activity against numerous fungi, against which echinocandins were not active. More specifically, the compounds according to the present invention are active against *Aspergillus* and *Candida* and *Fusarium* species, amongst others. Furthermore, due to the presence of two additional hydroxy groups and/or various carbohydrates in the molecule, the compounds according to the present invention are more hydrophilic than the echinocandins and therefore may have a higher bio-availability. This makes the compounds according to the present invention more suitable for oral application. Additionally, the compounds according to the present invention can for example be isolated from known cyanobacteria

These cyanobacteria are easy to cultivate in large quantities. Hence the compounds according to the present invention are cheap in their production. All the aforementioned advantages were not to be expected, and, therefore, make the compounds according to the present invention prime candidates, particularly suitable for pharmaceutical applications.

One of the members of this new class of compounds, hassallidin A (1) (shown below), a glycosylated lipopeptide, was isolated from an epilithic cyanobacterium collected in Bellano, Italy identified as *Tolypothrix* (basionym *Hassallia)* species, *Hassallia* sp. B07 applicant's reference sign (HAS B07). This cyanobacterium has been deposited by the applicants with the Deutsche Sammlung für Mikroorganismen und Z ellkulturen (DSMZ) in Braunschweig, Germany, under the applicant's reference sign HAS BO7 on February 22, 2005, and it has received the official DSMZ accession

no. DSM 17156. The compounds according to the present invention may however also be isolated from other publicly available cyanobacteria strains, in particular *Tolypothrix* strains, as outlined further below.

Chemical, mass spectrometric and spectroscopic analyses, including one- and two-dimensional NMR, were performed to determine an esterified 8 residue cyclic peptide linked with a carbohydrate and a fatty acid residue. Chiral GC-MS analysis revealed the occurrence of the non-proteinogenic amino acids D-*allo*-Thr, D-Thr, D-Tyr, D-Gln and dehydroaminobutyric acid (Dhb) within the peptide moiety. The additional components of hassallidin A could be identified as $\alpha,\beta$-dihydroxytetradecanoic acid (Dht) and mannose. The compounds according to the present invention, as exemplified by hassallidins A and B, exhibit antifungal activity against *Aspergillus fumigatus* and *Candida albicans* and other fungi with MIC values of 2-16 $\mu$g/mL for these test organisms.

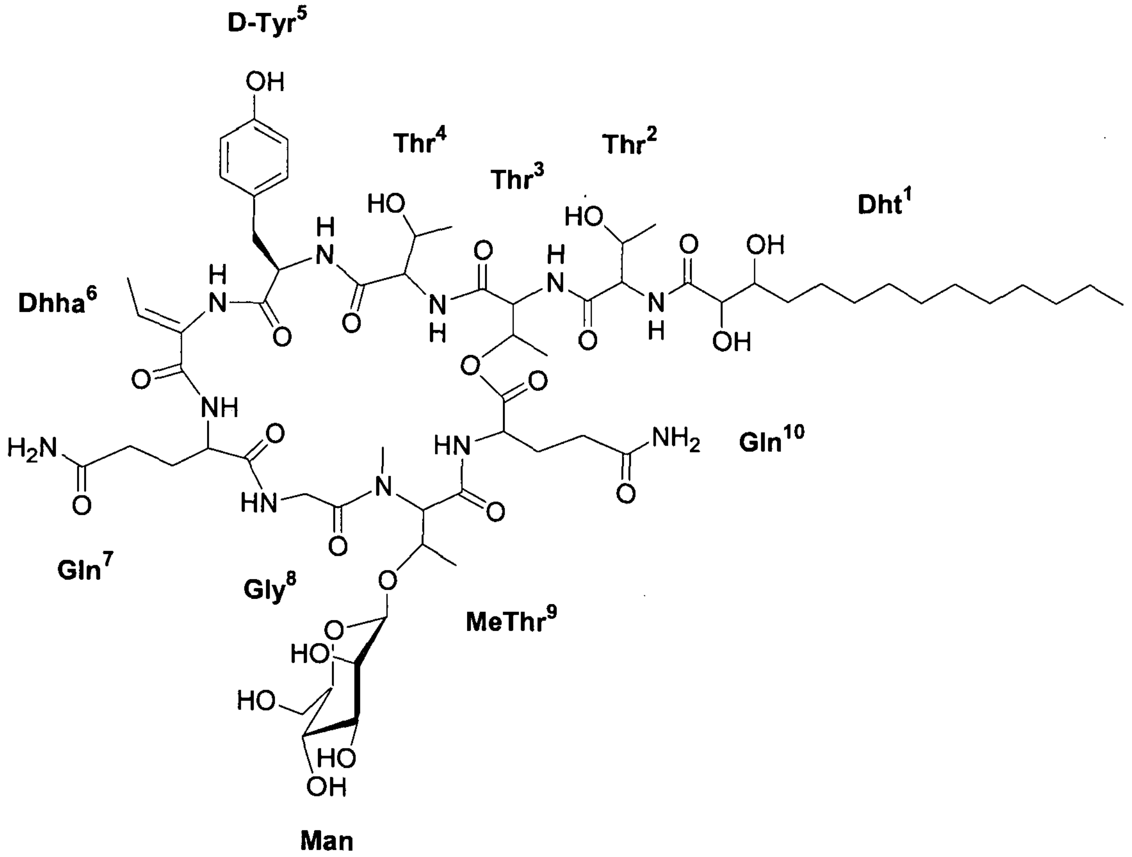

**1 hassallidin A**

In the following reference is made to the figures, wherein

fig. 1 s hows $^1H$-$^{15}N$-HSQC ofh assallidin A i n $d_6$-DMSO at 600 M Hz. Eight correlations from amino protons to the directly attached nitrogen are visible, labeled according to the amino acid. In addition, the $NH_2$ groups of the two glutamines yield four signals.

figure 2 shows $^1H$-$^{13}C$-DEPT-HMQC of hassallidin A in $d_6$-DMSO at 600 MHz. Correlations from all protons directly attached to carbon are visible, those of methylene groups are negative and displayed in gray. In the region of the aromatic resonances (> 110 ppm), the correlation of the tyrosine ring and the $\beta$-position of dehydroaminobutyric acid are visible. The anomeric carbon (C1) of the mannose is clearly visible at 96 ppm,

figure 3 shows a region of the $^1H$-$^{13}C$-HMBC (a) and the $^1H$-$^{13}C$-HMQC (b) of hassallidin A in $d_6$-DMSO at 600 MHz. In the HMBC correlations of the H1-proton within the mannose (anomeric proton) via $^2J_{HC}$ (reaching C2) and $^3J_{HC}$ (reaching C3 and C5) are visible. In addition the correlation to the C$\beta$ of MeThr9 proves the attachment of the mannose to the side chain of MeThr9 via its anomeric position,

figure 4 shows a region of the $^1H$-$^{13}C$-HMBC of hassallidin A in $d_6$-DMSO at 600 MHz. Correlations of the side chain carbonyl carbons of the two glutamines are visible as well as those of the carbonyl of the fatty acid (Dht1). The latter shows correlation to the OH and the proton attached to the second carbon atom in the fatty acid chain. More importantly, a correlation to the amino proton of Thr2 is visible, proving the attachment of Dht1 to the nitrogen of Thr2, and

figure 5 shows the fingerprint region from the NOESY of hassallidin A in $d_6$-DMSO at 600 MHz. Correlations from the amino protons to the $H^\alpha$ protons are visible. Intraresidue correlations are marked with rectangles. The dotted lines indicate the "sequential walk" linking the individual amino acids together. Two links are not depicted (Dhb6 to Gln7 and Gly8 to MeThr9) since the relevant correlations are outside of the spectral region shown here.

fig. 6 shows the differences in absorbance ($t_{0h}$-$t_{24h}$) of resazurin metabolised by L 929 cells, at different concentrations of hassallidin A and B, and $Na_2SeO_3$.

The following examples are given to illustrate the invention, not to limit the same.

**Examples**

**1. Isolation**

The investigated cyanobacterial species was isolated in 2002 from epilithic cyanobacteria collected in Orrido Clough, Bellano, Italy and identified as *Hassallia* sp. according to Geitler's characterization and taxonomy scheme.[8] *Hassallia* sp. B07 This cyanobacterium has been deposited with the DSMZ, Braunschweig, Germany, under the applicant's reference HAS B07 on February 22, 2005. It has received the official DSMZ accession no. DSM 17156. The compounds can, however, also be isolated from other *Tolypothrix* species publicly available, such as the following exemplary *Tolypothrix* strains, using essentially the same isolation protocol. Exemplary *Tolypothrix* strains are those strains deposited with the American Type Culture Collection as ATCC 20335, ATCC 27914, ATTC 29157, ATTC 29158, or those deposited with the Pasteur Culture Collection under the numbers PCC 6305, PCC 6601, PCC 7101, PCC 7415, PCC 7504, PCC 7601, PCC 7601/1, PCC 7708, PCC 7710, PCC 7712, PCC 7908, PCC 7910, PCC 9009.

Cells were grown for 30 days in modified BG-11 medium according to Welker[9] at 20 °C in 20 L flasks and were continuously illuminated and aerated. After filtration and lyophilisation, 4.3 g of dry material were obtained. The freeze-dried material was treated with MeOH to extract the active compound from the cyanobacterial biomass. After evaporation of solvent, the residue was eluted from a solid-phase extraction cartridge in preparation for an HPLC. The biologically active fractions, collected on eight reversed-phase HPLC runs with an $H_2O$/MeCN gradient, were combined to yield 2.4 mg of hassallidin A (1). The new compound, hassallidin A (1), was isolated as a white amorphous powder. High-resolution electrospray Fourier transform ion cyclotron mass spectrometry (ESI-FTICR-MS) of hassallidin A revealed a quasi-molecular ion $[M+Na]^+$ at *m/z* 1404.67572 consistent with the calculated molecular formula of $[C_{62}H_{99}N_{11}O_{24}\ Na]^+$, (requires m/z 1404. 67618, relative mass error $\Delta_m$= 0.33 ppm). The signals from the UV spectrum detected at $\lambda_{max}$ 226 nm ($\varepsilon$ 5500), 278 nm ($\varepsilon$ 880) and 265 nm ($\varepsilon$ 700) predicted the occurrence of possible aromatic amino acid residues within the hassallidin A structure. The IR spectrum showed a strong absorption at 1740 $cm^{-1}$, predicting the presence of an ester group (lactone) within the molecular structure as well.

Amino acid analysis and quantification by enantiomer labeling of the total hydrolysate and chiral g as c hromatography-mass spectrometry (GC-MS) gave the following relative molar concentrations: D-Tyr (1.00 ref.), D-Thr (0.96), L-Thr (0.88), D-*allo*-Thr (1.46), *N*-MeThr (1.01), D-Glu (0. 82), L-Glu (0.90), G ly (1.02), a nd d ehydroaminobutyric acid (Dhb) (one residue). Sugar analysis by GC-MS of the trimethylsilyl (TMS)-methylglycoside produced by acid hydrolyses and derivatisation revealed the presence of only mannose. Additionally, by GC-MS analysis the fatty acid was identified as dihydroxytetradecanoic acid (Dht) by commparison of its native mass with that of the corresponding TMS-methyl derivative. The $\alpha$- and $\beta$-position of the two hydroxy-groups were determined by two-dimensional NMR experiments.

**2. NMR-spectroscopy**

NMR spectroscopy was used to determine the constitution and arrangement of the individual "building blocks" independent from other methods. While a linear peptide of the size of hassallidin A would exhibit proton shifts near the random coil values, the good dispersion of the signals in the spectrum is in accordance with a cyclic peptide.

The analysis of the NMR data began with a comparison of the one-dimensional $^1H$-spectrum with a set of heteronuclear spectra. An $^1H$-$^{15}N$-correlation spectrum ($^{15}N$-HSQC) showed eight signals from secondary amide protons and two pairs of signals from primary amide groups (Figure 1). An $^1H$-$^{13}C$-correlation spectrum ($^{13}C$- DEPT-HMQC, Figure 2) showed three signals in the range between 6 ppm and 10 ppm as the only carbon-bound protons in that region.

The integral of the broad proton signals around 9.17 ppm indicated two protons close to each other. Only the signal at 9.18 ppm was found in the $^1H$-$^{15}N$-correlation. The signal at 9.16 ppm therefore indicated a proton neither bound to $^{15}N$ nor to $^{13}C$, most likely an OH at the aromatic ring of tyrosine.

Further analysis of the carbon bound protons was based on the HMQC and in addition on a DEPT-HMQC and an HQQC. The latter showed only signals from methyl groups while the former indicated the different multiplicities of the carbon spectra by an inverted sign for methylene groups. Seven methyl groups could be detected, one of them at chemical shifts of 2.99 ppm ($^1H$) and 30.64 ppm ($^{13}C$), which are typical values for *N*-methyl groups. In addition 18 CH groups were found, one with a chemical shift of 95.95 ppm, typical for anomeric signals of hexose moieties. The remaining 11 signals were from $CH_2$ groups. Some of those signals, however, clearly represented more than one carbon atom. In particular, the region between 1.15 ppm and 1.5 ppm ($^1H$ chemical shift) showed five $CH_2$ and one $CH_3$ group (see insert in Figure 2). The integration of the one-dimensional spectrum, however, indicated the presence of 23 protons. The strong peak at 1.23 ppm/28.75 ppm thus had to contain six $CH_2$ groups, which was confirmed by inspecting a one-dimensional carbon spectrum. Since no individual assignment is possible all carbon are given with the same chemical shift in Table 1. Several signals from the one-dimensional $^1H$ spectrum had no corresponding signals in the HMQC and were thus most likely OH protons, since no exchange broadening was observable.

The next step of the identification of individual residues in hassallidin A focused on proteinogenic amino acids. The analysis was initially based on heteronuclear spectra: The HMQC was combined with an HMQC-COSY and HMQC-TOCSYs with different mixing times as well as an HMBC. The results obtained from those spectra were subsequently confirmed by using homonuclear spectra, in particular a DQF-COSY and TOCSY spectra of several mixing times. Based on those spectra, the following amino acids could be identified: glycine, tyrosine, two glutamines, and three threonines. Additional signals typical for another threonine were also found, but the amino proton was lacking. A correlation of the methyl group at 2.99/30.64 ppm ($^1H/^{13}C$) to the $C^\alpha$ of that threonine in the HMBC confirmed that the fourth threonine was methylated at the nitrogen. Two of the threonines did not show signals of OH protons. Since all other OH protons were visible, these two threonine side chain were most likely chemically modified.

The next step was the assignment of the remaining resonances to moieties other than proteinogenic amino acids. The spectra indicated that the signal at 6.41/130.12 ppm ($^1H/^{13}C$) was coupled to one of the methyl groups, because no other protons seemed to belong to the same spin system. Based on correlations found in the HMBC, the signals could be linked to the amino proton at 9.18 ppm that did not show any correlation in homonuclear spectra and to a further, quaternary carbon at 129.72 ppm. In conjunction, the signals added up to a dehydroaminobutyric acid residue (Dhb), which could subsequently be confirmed in a homonuclear NOESY spectrum. Since this spectrum showed a correlation from the amino proton to the methyl group but not to the olefinic proton, Dhb had to be in Z-configuration.

Resonances in the region between 60 and 75 ppm in the $^{13}C$ NMR spectrum and around 3.5 ppm in the $^1H$ NMR spectrum were all identified as signals from a hexose moiety. Correlations to the resonance at 95.95 ppm in carbon were also found. The type of hexose could not be determined from NMR spectra. All protons of the hexose showed correlations to OH protons in the HMQC-COSY spectrum except for the anomeric carbon. This indicated that the hexose is linked to the peptide via its anomeric carbon. Figure 3 shows a region of the HMBC. The proton attached to the anomeric carbon of the hexose ($H1^{Man}$) showed a correlation to the $C\beta$ of Thr9 in the HMBC, thus confirming the link of the hexose to the threonine that was previously identified as an *N*-methylated threonine.

Two correlations in the HMQC, at 3.81/73.05 ppm ($^1H/^{13}C$) and 3.65/71.09 ppm ($^1H/^{13}C$), showed correlations to OH protons and a coupling between each other in the HMQC-COSY. The signal at 3.65/71.09 ppm ($^1H/^{13}C$) showed a correlation to the bulk of $CH_2$ groups around 1.3 ppm. HMQC-TOCSY and HMBC spectra exhibited many correlations within these carbons and to the methyl group at 0.84/13.65 ppm ($^1H/^{13}C$). Altogether this indicated the presence of a long chain of 13 protonated carbon atoms, the first two having an OH group attached, the final one being a methyl group. Figure 4 shows a region of the HMBC with correlations from the proton attached to the first carbon of that long chain, the OH attached to it and the amino proton of Thr2 all attached to the same carbonyl carbon at 176.5 ppm. This indicates that the carbonyl carbon is the first carbon in a $C_{14}$, $\alpha,\beta$-dihydroxytetradecanoic acid chain (Dht) that is attached to the nitrogen of the first threonine in the chain.

Since the HMBC did not show enough correlations from amino protons to carbonyl carbons, the amino acid sequence was established based on sequence specific assignment using NOESY and TOCSY spectra. Intraresidue peaks in the NOESY were identified by comparison with the TOCSY spectrum. Then a "sequential walk" was performed as shown in Figure 5. Correlations between amino protons and both $H^\alpha$ and $H^\beta$ protons confirmed the sequential connectivities. Partial sequences could thus be established: the first was Thr-Thr-Thr-Tyr-Dhb, the second Gln-Gly and the third MeThr-Gln. Between the three chains, the missing $H^\alpha$ in Dhb and the missing amino proton in MeThr prevented use of the conventional strategy. Sequential correlations between amino proton of Gln7 and the $H^\beta$ proton of Dhb as well as the *N*-methyl group of MeThr9 and the $H^\alpha$ protons of Gly8, however, closed the two gaps. A sequence Thr-Thr-Thr-Tyr-Dhb-Gln-Gly-MeThr-Gln could therefore be established. The remaining question was the missing OH proton of Thr3. Since the peptide is obviously cyclised via a lactonic bond, a cyclization via the side chain of Thr3 is likely. A complete list of all $^1H$, $^{13}C$ and $^{15}N$ chemical shifts and the assignment of hassallidin A is given in Table 1.

**Table 1.** $^{1}H$ and $^{13}C/^{15}N$ NMR data for hassallidin A in $d_6$-DMSO

| component | position | $\delta_H$ (ppm) | $\delta_{C/N}$ (ppm) |
|---|---|---|---|
| Dht-1 | 14 | 0.84 | 13.64 |
| | 13 | 1.25 | 21.80 |
| | 12 | 1.23 | 30.96 |
| | 11[a] | 1.23 | 28.75 |
| | 10[a] | 1.23 | 28.75 |
| | 9[a] | 1.23 | 28.75 |
| | 8[a] | 1.23 | 28.75 |
| | 7[a] | 1.23 | 28.75 |
| | 6[a] | 1.23 | 28.75 |
| | 5 | 1.35/1.22 | 25.11 |
| | 4 | 1.42 | 32.41 |
| | 3 | 3.65 | 71.11 |
| | 2 | 3.81 | 73.47 |
| | 2-OH | 5.49 | |
| | 3-OH | 4.38 | |
| | CO | | 176.50 |
| Thr-2 | HN | 7.62 | 108.67 |
| | α | 4.33 | 56.93 |
| | β | 4.11 | 66.09 |
| | γ | 1.03 | 19.48 |
| | OH | 5.12 | |
| | CO | | 169.88 |
| Thr-3 | HN | 7.98 | 111.33 |
| | α | 4.54 | 54.77 |
| | β | 4.91 | 70.64 |
| | γ | 1.11 | 15.47 |
| | CO | | n.d. |
| Thr-4 | HN | 7.78 | 117.09 |
| | α | 4.21 | 58.28 |
| | β | 3.95 | 66.56 |
| | γ | 1.09 | 20.08 |
| | OH | 5.20 | |
| | CO | | n.d. |
| Tyr-5 | HN | 8.64 | 125.58 |
| | α | 4.37 | 55.24 |
| | β | 2.90/2.83 | 35.50 |
| | 1 | | 126.96 |
| | 2/6 | 7.02 | 130.12 |
| | 3/5 | 6.63 | 115.00 |
| | 4 | | 155.92 |
| | OH | 9.16 | |
| | CO | | 170.22 |
| Dhb-6 | HN | 9.18 | 120.60 |
| | α | | 129.72 |
| | β | 6.41 | 129.65 |
| | γ | 1.23 | 12.41 |
| | CO | | 163.37 |
| Gln-7 | HN | 8.10 | 119.11 |
| | α | 3.94 | 52.41 |

(continued)

| component | position | $\delta_H$ (ppm) | $\delta_{C/N}$ (ppm) |
|---|---|---|---|
| | β | 1.97/1.90 | 25.78 |
| | γ | 2.13 | 31.03 |
| | CO | | n.d. |
| | γ-CO | | 174.49 |
| | $NH_2$ | 7.31/6.80 | 109.72 |
| Gly-8 | HN | 8.40 | 107.41 |
| | α | 4.11/3.93 | 40.37 |
| | CO | | 169.88 |
| MeThr-9 | $NCH_3$ | 2.99 | 30.64 |
| | α | 4.79 | 59.96 |
| | β | 4.23 | 67.10 |
| | γ | 1.08 | 14.79 |
| | CO | | n.d. |
| Man | 1 | 4.77 | 95.95 |
| | 2 | 3.52 | 70.34 |
| | 3 | 3.36 | 70.47 |
| | 4 | 3.43 | 66.16 |
| | 5 | 3.34 | 73.84 |
| | 6 | 3.45/3.58 | 60.57 |
| | 2-OH | 4.67 | |
| | 3-OH | 4.49 | |
| | 4-OH | 4.62 | |
| | 6-OH | 4.24 | |
| G1n-10 | HN | 7.83 | 113.36 |
| | α | 4.38 | 52.54 |
| | β | 1.87 | 28.07 |
| | γ | 2.08/2.13 | 31.03 |
| | CO | | n.d. |
| | γ-CO | | 174.22 |
| | $NH_2$ | 7.30/6.86 | 109.73 |

[α] Due to overlap in the NMR, spectra cannot be individually assigned

### 3. Mass spectrometry

Deduced data of the hassallidin A constitution obtained by NMR and acid hydrolysis were confirmed by MALDI-TOF post-source decay (PSD) and ESI-$MS^{(n)}$ experiments. The reflector mode MALDI-TOF mass spectrum performed with delayed extraction (DE) showed a positive i on signal at m /z 1404.8, identified as a s odium-ion associated monoisotopic peak $[M+Na]^+$ of hassallidin A. In addition there was a second significant mass signal at m/z 1220.7 in the reflector mode, corresponding to a protonated mass signal of hassallidin A without mannose (mass loss of m/z 162). Additional signals in the reflector mode during analysis of glycopeptides are caused by a rapid formation of ions within the ion source, which is known as in-source decay (ISD).[10] Influenced by different parameters, like matrix, compound residues, and the use of delayed extraction, [11] ISD fragments can often be observed in the same spectrum when recorded with delayed extraction TOF instruments operated in the reflector mode. By the use of the ISD ion as precursor ion, in the PSD mode, subsequence information of tthe l ipopeptide moiety confirmed the predicted amino a cid sequence of the NMR experiments. Interpretations of fragment mass signals are listed in Table 2. By using the collision cell in the low mass range of the PSD mode, immonium ions ($^+NH_2$=CH-R) were released to provide further information about the present amino acids. Immonium ion signals (rel. int. %) for Thr *m*/*z* 74 (14), MeThr *m*/*z* 88 (20), Gln *m*/*z* 101 (13), Gly *m*/*z* 30 (2), Tyr *m*/*z* 136 (4), and Dhb *m*/*z* 56 (6) were detected.

**Table 2**. MALDI-TOF-PSD fragment-ions of the hassallidin A lipopeptide moiety

| ion composition[b] | (m/z)/int.(%) |
|---|---|
| M - [*m/z* 243 (Dht)] | 977/ (2) |
| M - [*m/z* 243 (Dht)-*m/z* 101 (Thr)] | 876/ (8) |
| M - [*m/z* 128 (Gln)] | 1092/ (11) |
| M - [*m/z* 128 (Gln)-*m/z* 57 (Gly)] | 1035/ (5) |
| M - [*m/z* 128 (Gln)-*m/z* 57 (Gly)-*m/z* 115 (MeThr)] | 920/ (33) |
| M - [*m/z* 128 (Gln)-*m/z* 57 (Gly)-*m/z* 115 (MeThr)-*m/z* 128 (Gln)] | 792/ (7) |
| M - [*m/z* 83 (Dhb)-*m/z* 128 (Gln)-*m/z* 57 (Gly)-*m/z* 115 (MeThr)-*m/z* 128 (Gln)] | 709/ (2) |
| M - [*m/z* 163 (Tyr)-*m/z* 83 (Dhb)-*m/z* 128 (Gln)-*m/z* 57 (Gly)-*m/z* (MeThr)-m/z 128 (Gln)] | 115546/ (3) |
| M - [m/z 101 (Thr)-m/z 163 (Tyr)-m/z 83 (Dhb)-m/z 128 (Gln)-m/z (Gly)-m/z 115 (MeThr)-m/z 128 (Gln)] | 57445/ (6) |
| [*m/z* 57 (Gly)-m/z 115 (MeThr)+H]$^+$ | 173/ (33) |
| [m/z 83 (Dhb)-m/z 128 (Gln)+H]$^+$ | 212/ (4) |
| [m/z 57 (Gly)-m/z 115 (MeThr)-m/z 128 (Gln)+H] $^+$ | 301/ (48) |
| [m/z 57 (Gly)-m/z 115 (MeThr)-m/z 128 (Gln)-m/z 83 (Dhb)+H]$^+$ | 384/ (38) |
| [m/z 128 (Gln)-m/z 57 (Gly)-m/z 115 (MeThr)-m/z 128 (Gln)+H]$^+$ | 429/ (21) |

[b]*M* = [*m/z* 1381+H]$^+$- mannose [m/z 162] = [m/z 1219+H]$^+$

Fragmentation patterns of the protonated monoisotopic peak at m/z 1382 for hassallidin A were also studied using ESI-MS/MS experiments. Formation of one first-generation fragment ion was accompanied by a loss of *m/z* 162 for mannose to result in a mass signal of *m/z* 1220. The following daughter-fragment ion series of the first generation ion M were detected and interpreted as: *m/z* 1092: M- [*m/z* 128 (Gln)], *m/z* 920: M- [*m/z* 128 (Gln)-*m/z* 57 (Gly)-*m/z* 115 (MeThr)] and *m/z* 792: M- [*m/z* 128 (Gln)-*m/z* 57 (Gly)-*m/z* 115 (MeThr)-*m/z* 128 (Gln)].

4. **Antifungal activity**

The antifungal activity of the compounds according to the present invention, in particular hassallidin A and hassallidin B, was verified for *Aspergillus fumigatus* and *Candida albicans* by minimum inhibitory concentration (MIC) data in a serial dilution test. For each fungus six testing wells of a microtiter plate were prepared with 240 μL medium (Sigma RPMI-1640), 10 μL inoculum suspension and 50 μL fungicide solution (50% MeOH) of different concentrations. Inoculum preparation was carried out by guideline of NCCLS.[25] The fungicide solution preparation took place in a series of twofold dilutions from a stock solution of 92 μg/mL starting with 4.6 μg/50 μL down to 0.14 μg/50 μL. One well was prepared without antifungal agent for unhindered growth as control. The microtiter plates were incubated for *Candida albicans* and *Aspergillus fumigatus* at 35 °C for 24 h and 48 h, respectively. Tests were run in triplicates. Inhibition of growth was assessed by comparing the fungal growth of the test samples with the control well by determination of optical density at 595 nm in a microplate reader. The concentration of the well at highest dilution that was still free from growth was assigned the minimum inhibitory concentration (MIC in μg/mL). MIC values for hassallidin A and hassallidin B against *Candida albicans* and *Aspergillus fumigatus* were found to be 4.8 μg/mL for both test organisms, using RPMI-1640 medium. Hassallidin A and hassallidin B also shows precise antifungal activity when tested in disk diffusion assays (10 μg in 30 μL 50% MeOH per disk) on malt agar plates against *Aspergillus fumigatus, Aspergillus niger, Ustilago maydis, Penicillium* sp., *Fusarium sambucium, Candida albicans,* and *Candida glabrata.* No activities were observed by using *Bacillus subtilis, Streptomyces versicolor* or *Escherchia coli* in disk diffusion assays on LB-agar plates with 10 μg hassallidin A in 30 μL 50% MeOH per disk.

More specifically, a determination of inhibitory concentration (IC) of hassallidin A and hassallidin B for various fungi, *Candida* species and *Candida* strains were performed with different media (RPMI-1640 or SAB or AM3) in a microdilution assay by following the guidelines in NCCLS documents M[38]-P[25] and M27-A2[26] carried out as follows
Each substance was dissolved with 100% dimethyl sulfoxide (Serva, Heidelberg, Germany) to give a stock solution of 3.2 mg/ml followed by nine log2 dilution steps with the solvent.
This served as a 100-fold concentrated dilution series ranging from 1.6 mg/ml to 0.003 mg/ml of the test substance. The final concentration of the test substance was 16 μg/ml to 0.031 μg/ml.

Therefore the 100-fold concentrated dilution series (with 100% DMSO) was diluted to the final concentration by two dilution steps:

(1) Each concentration of the 100-fold concentrated substance was diluted with test medium for 1:50 (for ex.: adding

of 13 μl of the test substance to 637 μl test medium).

(2) 100 μl of each 1:50 diluted concentration was filled in a row of wells of a microtitre plate. One well was without the substance but with the 1:50 diluted solvent serving as growth control.

A further 1:2 dilution of the antifungal was made by the inoculation of each well with 100 μl of the test organism, which was suspended in the test medium.

The remaining well of the row was used for sterility control of the medium.

The inoculum was strictly prepared according the NCCLS[25] guidelines, resulting in 0.5-2.5 CFU's/ml for *Candida-species* and *Cryptococcus neoformans.* For hyphomycetes CFU's were tenfold higher (see NCCLS). [26]

The growth of the hyphomycetes respectively the inhibition was measured by visual reading with the help of a reading mirror; scoring was done according to NCCLS :[26] score 0 corresponds to a optical clear well or an $IC_{95}$ or higher, score 1 corresponds to an inhibition of 80 percent ($IC_{80}$) and the score 2 of the NCCLS definition corresponds to the $IC_{50}$ values in the tables. The growth of the yeasts was measured spectrophotometrically by a microplate reader (MR 5000, Dynatech laboratories) at 630 nm displaying the optical density (OD). The percent inhibition $IC_{50}$, $IC_{80}$ or $IC_{95}$ (corresponding the scores of 2, 1 and 0) was calculated by the following formula:

$$\frac{100 - [\ (\text{OD of the well} - \text{OD of the medium})]\ \times 100}{(\text{OD of the growth control} - \text{OD of the medium}).}$$

**Table 3.** Test series for determining IC values of hassallidin A and B in different media using *Candida albicans* and *Candida krusei.* The IC values were measured after 24 h and 48 h at 30 °C.

| | | RPMI | RPMI | RPMI | RPMI | SAB | SAB | SAB | SAB | AM3 | AM3 | AM3 | AM3 |
|---|---|---|---|---|---|---|---|---|---|---|---|---|---|
| | | **24h** | **48h** | **24h** | **48h** | **24h** | **48h** | **24h** | **48h** | **24h** | **48h** | **24h** | **48h** |
| **strain** | **IC μg/mL** | **HA A** | **HA A** | **HA B** | **HA B** | **HA A** | **HA A** | **HA B** | **HA B** | **HA A** | **HA A** | **HA B** | **HA B** |
| ATCC 6258 (QC) | $IC_{50}$ | 16 | 16 | 16 | 16 | n.d. | n.d. | n.d. | n.d. | n.d. | n.d. | n.d. | n.d. |
| ATCC 6258 (QC) | $IC_{80}$ | 16 | 16 | 16 | 16 | n.d. | n.d. | n.d. | n.d. | n.d. | n.d. | n.d. | n.d. |
| ATCC 6258 (QC) | $IC_{95}$ | 16 | 16 | 16 | 16 | n.d. | n.d. | n.d. | n.d. | n.d. | n.d. | n.d. | n.d. |
| SC 5314 | $IC_{50}$ | 16 | 16 | 16 | 16 | 4 | 8 | 8 | 8 | 4 | 8 | 4 | 8 |
| SC 5314 | $IC_{80}$ | 16 | 16 | 16 | 16 | 4 | 8 | 8 | 8 | 4 | 8 | 4 | 8 |
| SC 5314 | $IC_{95}$ | 16 | 16 | 16 | 16 | 4 | 8 | 8 | 8 | 4 | 8 | 4 | 8 |

HA A = Hassallidin A
HA B = Hassallidin B
ATCC 6258 (QC) = Quality control strain *(Candida krusei)*
SC 5314 = *Candida albicans*
RPMI, SAB, AM3 = media (composition, see "6. General Experimental Section")
n.d. = not determined

**Table 4.** Further test series, IC values for hassallidin A in RPMI-1640 medium were determined for various fungi, *Candida* species and *Candida* strains. The IC values were measured after 24 h and 48 h at 30 °C and 35 °C.

| strain | IC (μg/mL) | 35°C/24h | 35°C/48h | 30°C/24h | 30°C/48h |
|---|---|---|---|---|---|
| *Candida albicans* ATCC 90028 | $IC_{50}$ | 8 | 8 | | |
| *Candida albicans* ATCC 90028 | $IC_{80}$ | 8 | 8 | | |
| *Candida albicans* ATCC 90028 | $IC_{95}$ | 8 | 8 | | |
| ATCC 6258 (QC) | $IC_{50}$ | 8 | 16 | 8 | 8 |
| ATCC 6258 (QC) | $IC_{80}$ | 8 | 16 | 8 | 8 |
| ATCC 6258 (QC) | $IC_{95}$ | 8 | 16 | 8 | 8 |
| *Candida albicans* SC 5314 | $IC_{50}$ | 8 | 8 | | |
| *Candida albicans* SC 5314 | $IC_{80}$ | 8 | 8 | | |
| *Candida albicans* SC 5314 | $IC_{95}$ | 8 | 8 | | |
| *Candida* spp. I | $IC_{50}$ | 8 | 8 | | |
| *Candida spp.* I | $IC_{80}$ | 8 | 8 | | |
| *Candida spp.* I | $IC_{95}$ | 8 | 8 | | |
| *Candida spp.* II | $IC_{50}$ | 8 | 8 | | |
| *Candida spp.* II | $IC_{80}$ | 8 | 8 | | |
| *Candida spp.* II | $IC_{95}$ | 8 | 8 | | |
| *Cryptococcus neoformas* RKI 18/05 | $IC_{50}$ | 4 (visual) | 4 | | |
| *Cryptococcus neoformas* RKI 18/05 | $IC_{80}$ | 4 (visual) | 4 | | |
| *Cryptococcus neoformas* RKI 18/05<br>*Aspergillus fumigatus* RKI 584/00 | $IC_{95}$<br>$IC_{50}$ | 4 (visual)<br>2 | 4 | | |
| *Aspergillus fumigatus* RKI 584/00 | $IC_{80}$ | 2 | 4 | | |
| *Aspergillus fumigatus* RKI 584/00 | $IC_{95}$ | 2 | 4 | | |
| *Aspergillus fumigatus* RKI 914/02 | $IC_{50}$ | 2 | 2 | | |
| *Aspergillus fumigatus* RKI 914/02 | $IC_{80}$ | 2 | 2 | | |
| *Aspergillus fumigatus* RKI 914/02 | $IC_{95}$ | 2 | 4 | | |
| *Fusarium solani* RKI 144/03 | $IC_{50}$ | | | 16 | >16 |
| *Fusarium solani* RKI 144/03 | $IC_{80}$ | | | 16 | >16 |
| *Fusarium solani* RKI 144/03 | $IC_{95}$ | | | >16 | >16 |
| *Fusarium solani* RKI 233/04 | $IC_{50}$ | | | 16 | >16 |
| *Fusarium solani* RKI 233/04 | $IC_{80}$ | | | 16 | >16 |
| *Fusarium solani* RKI 233/04 | $IC_{95}$ | | | >16 | >16 |
| *Acremonium strictum* RKI 226/03<br>*Acremonium strictum* RKI 226/03 | $IC_{50}$<br>$IC_{80}$ | | | 4-8<br>16 | >16<br>> 16 |
| *Acremonium strictum* RKI 226/03 | $IC_{95}$ | | | 16 | > 16 |
| ATCC 6258 (QC) = Quality control strain *(Candida krusei)* | | | | | |

**5. Activity against cancerous cells**

Furthermore, the compounds according to the present invention were tested in relation to their activity against

cancer cell lines. They were tested for cytotoxicity by determining the $IC_{50}$-values. The $IC_{50}$-value was determined by the procedure discrided in Filip P, Anke T, Sterner O, 5-(2'-oxoheptadecyl)-resorcinol and 5-(2'-oxononadecyl)-resorcinol, cytotoxic metabolites from a wood-inhabiting basidiomycete, *ZNaturforsch* [C]. 2002 Nov-Dec;57(11-12):1004-8. Sample preparation was carried out as follows:

1 st test

The compound (hassalidin B) (20 μg) was dissolved in 4 μl DMSO and 16 μl $H_2O$ to give a concentration of 1 mg/ml. 1 μl of this solution and 0,5 μl of this solution were used to inoculate 200 μl culture medium each, thus making up a concentration of hassallidin B of 5μg/ml and 2,5 μ/ml, respectively. Thereafter 68 μl $H_2O$ were added to 17 μl of the solution of 1 mg/ml hassalidin B to give a concentration of 200 μg/ml, and again 1 μl and 0,5 μl of this solution were used to inoculate 200 μl of culture medium each thus making up a final concentration of hassallidin B of 1 μg/ml and 0,5 μg/ml, respectively. The 200μg/ml solution was diluted 1:5-fold with $H_2O$ to give a concentration of 40 μg/ml, and again 1 μl and 0,5 μl were used in 200 μl culture medium each, thus making up a concentration of 0,2 μg/ml and 0,1 μg/ml, respectively. All concentrations were performed in duplicates.

Cell viability of cell growing was measured by the XTT test (Boehringer, Mannheim/Roche) as discribed in the product information.

Using the Jurkat ATCC-TIB-152 (human acute T cell leukemia) celline an $IC_{50}$-value of 0.2 μg/ml could be determined.

**Table 5.** IC values of hassallidin B using Jurkat ATCC-TIB-152 celline

| Hassallidin B | 5 μg/ml | 2,5 μg/ml | 1μg/ml | 0,5 μ/ml | 0,2 μg/ml | 0,1 μg/ml |
|---|---|---|---|---|---|---|
| Activity | +/+ | +/+ | +/+ | +/+ | +/-/- | -/- |
| + = > 80% bleak cells<br>+/- = 30-80% bleak cells<br>- = no cytotoxic effect | | | | | | |

2nd test

Cytotoxicity assays were further performed using the IC-Tox50 Kit from CCS (Cell Culture Service, Hamburg) according to the manufacturers recommendations in 96-well microtitre plates. L 929 (murine aneuploid fibrosarcoma) cells were used and the activity was followed by recording the decrease of the blue pigment resazurin at 595 nm. Down to a concentration of 1.0 μg/mL a hundred percent inhibition of cell activity was determined for hassallidin A and hassallidin B. The cytotoxity assays using deposited L 929 cells were prepared as follows:

Each well contained 140 μl RPMI-1640 medium, 100 μl resazurin solution and 10 μl test solution of hassallidin A or hassallidin B at different concentrations. To obtain 10 μl test solution, 1 μl / 2 μl / 3 μl or 4μl of a 50% methanolic stock solution (25 μg/ 100 μl) were mixed with 9 μl / 8 μl / 7 μl or 6 μl 50% MeOH. For unhindered cell activity, 10 μl 50% MeOH solution was used as test solution. Solutions of 1μg $Na_2SeO_3$ / 10 μl (50% MeOH) and 2 μg $Na_2SeO_3$ /10 μl (50% MeOH) were used as positive control.

The absorption difference ($t_{0h}$-$t_{24h}$) of resazurin metabolised by L 929 cells is graphically represented in figure 6.

### 6. General Experimental Section

UV spectra were recorded on a Shimadzu UV-160 spectrophotometer. The IR spectra were recorded using a Bruker IFS 66v/S Fourier Transform Infrared spectrometer (FTIR).

NMR spectra were recorded at 600 MHz ($^1H$ frequency) using Bruker DRX spectrometers. One mg of hassallidin A was dissolved in 550 μL $d_6$-DMSO yielding a concentration of 1.4 mM. Most spectra were recorded using a 5 mm triple resonance probe (H,C,N) equipped with three-axis self-shielded gradients. A carbon one-dimensional spectrum was recorded using a 5 mm dual resonance probe (H,C). The $^{13}C$-HMBC and the $^{15}N$-HSQC were recorded using a cryogenic 5 mm triple resonance probe (H,C,N) equipped with one-axis self-shielded gradients. One-dimensional proton and carbon spectra were recorded with 32 and 100000 scans using 8k and 128k data points, respectively. All homonuclear two-dimensional spectra (DQF-COSY,[12] NOESY,[13] TOCSY[14,15]) were recorded using 2048 x 512 complex data points. The DQF-COSY was recorded using 32 scans; the TOCSYs were recorded using 16 scans and mixing times of 30, 60 and 120 msec; and the NOESY was recorded using 32 scans and a mixing time of 100 msec. Most heteronuclear two-dimensional spectra were recorded using 512 x 256 complex data points. The $^{13}C$-HMQC[16] and the $^{13}C$-DEPT-HMQC[17] were recorded using 96 scans, the $^{13}C$-HMQC-TOCSY[18] spectra were recorded with 160 and 256 scans using mixing times of 20 and 120 msec, respectively. The $^{13}C$-HMQC-COSY[19] was recorded with 512 x 384 complex data points using 512 scans. The $^{13}C$-HQQC[20] was recorded with 512 x 64 complex data points using a reduced spectral width and 96 scans. All the above heteronuclear spectra w ere recorded u sing a BIRD pulse for suppression of protons bound t

o $^{12}C$.[21] A gradient-$^{13}C$-HMBC[22] was recorded with 2048 x 512 complex data points using 176 scans, the $^{15}N$-HSQC[23] was recorded with 512 x 64 complex data points using 360 scans.

The GC-MS analyses were performed on an Agilent 6890/5793 MSD system. Mass spectra were obtained on a PerSeptive Biosystems Voyager-DE PRO MALDI-TOF mass spectrometer and on a Bruker esquire 2000 LC-MS system equipped with an electrospray source. The high-resolution ESI-FTICR mass spectrum was obtained on a Finnigan ThermoQuest device. HPLC separations were performed on a Waters 515 system coupled to photodiode array detector and autosampler. The optical density was measured with a Tecan-Genios microplate reader at 595 nm.

Media: The media RPMI-1640, SAB and AM3 had the following compositions The composition of medium RPMI-1640 is shown in table 6.

**Table 6.** Composition ofRPMI-1640 medium

| Constituent | g/L Water | Constituent | g/L Water |
|---|---|---|---|
| L-arginine (free base) | 0.200 | Biotin | 0.0002 |
| L-aspargine (anhydrous) | 0.050 | D-pantothenic | 0.00025 |
| L-aspartic acid | 0.020 | Cholint chloride | 0.003 |
| L.cystine•2HCl | 0.0652 | Folic acid | 0.001 |
| L-glutamic acid | 0.020 | Myo-inositol | 0.035 |
| L-glutamine | 0.300 | Niacinamide | 0.001 |
| Glycine | 0:010 | PABA | 0.001 |
| L-histidine (free base) | 0.015 | Pyridoxine HCl | 0.001 |
| L-hydroxyproline | 0.020 | Riboflavin | 0.0002 0.00005 |
| L-isoleucine | 0.050 | Thiamme HCl | 0.001 |
| L-leucine | 0.050 | Vitamin $B_{12}$ | 0.000005 |
| L- lysine · HCl | 0.040 | Calcium nitrate. $H_2O$ | 0.100 |
| L-methionine | 0.015 | Potassium chloride | 0.400 |
| L-phenylalanine | 0.015 | Magnesium sulfate (anhydrous) | 0.04854 |
| L-proline | 0.020 | Sodium chloride | 6.000 |
| L-serine | 0.030 | Sodium phosphate dibasic (anhydrous) | 0.800 |
| L-threonine | 0;020 | D-glucose | 2.000 |
| L-tryptophan | 0.005 | Glutathione; reduced | 0.001 |
| L-tyrosine • 2Na | 0.02883 | Phenol red, Na | 0.0053 |
| L-valine- | 0.020 | | |

RPMI-1640 medium was bufferd with MOPS (3-[N Morpholino] propanesulfonic acid) 0.165 M and the pH was adjusted to pH = 7.0 at 25°C.

SAB and AM3 media had the following composition:

AM3: Bacto-beef extract (1.5 g/L), bacto-yeast extract (1.5 g/L), bacto-peptone (5 g/L), bacto-dextrose (1 g/L), sodium chloride (3.5 g/L), dipotassium phosphate (3.68 g/L) and monopotassium phosphate (1.32 g/L), pH = 7.0 at 25°C.

SAB:1% bacto-peptone (w/v), 2% glucose (w/v), pH not adjusted.

Organism and Culture Conditions. *Hassallia* sp. BO7 (deposited under applicant's reference sign HAS B07 with DMSZ, Braunschweig, Germany on February 22, 2005, and now having official DSMZ accession no. DSM17156) was isolated in 2002 from epilithic cyanobacteria, which were collected in Orrido Clough, Bellano, Italy. The sample was preincubated in BG-11 medium modified by Welker:[9] [$NaNO_3$ (10 mM), $K_2HPO_4$ (1 mM), $MgSO_4$ x $7H_2O$ (0.7 mM), $CaCl_2$ (0.2 mM), $Na_2CO_3$ (0.2 mM), $Na_2EDTA$ (0.1 mM), citric acid (0.1 mM), $FeCl_3$ x $6H_2O$ (0.02 mM) containing 1mL/L medium trace elements additional with thiamine HCl (300 nM), biotin (2 nM), and cyanocobalamin (0.4 nM)] and individualized by plating cultural solution on agar plates (1% agarose in BG-11 medium). For mass cultivation the strain grew in 20 L polycarbonate bottles with the modified BG-11 medium and permanent sterile aeration at room temperature. The cultures were constantly illuminated by daylight lamps (Philips TLD 58W/840). Cyanobacterial material was harvested by percolation after 30 days, to give yields of lyophilised cells from 4.3 g.

Extraction and Isolation. A portion of 4 g freeze-dried cyanobacterial biomass was extracted with 75% MeOH (500 mL) for 3 h and was separated by centrifugation. After removal of solvent in a vacuum rotary evaporator at 50 °C, the crude extract (16 mg) was taken up in 20 ml 90% MeOH and eluted from a solid-phase extraction cartridge (Waters Oasis HLB Extraction Cartridge) with 75% MeOH, filtrated through 0.22 μm filter (Roth Rotilabo) and subjected to HPLC in eight portions [Waters Spherisorb S5 ODS2, 10 x 250 mm column; mobile phase: solvent A: $H_2O$/formic acid (0.05%), solvent B: acetonitrile/formic acid (0.05%); 3 mL/min; UV detection at 220 nm]. The following gradient was applied: solvent B from 30% to 35% in 10 min, 35% to 70% in 30 min, 70% to 100% in 4 min, isocratic 6 min. The fractions that exhibited antifungal activity eluted at 23 - 24 min. Fractions from eight HPLC runs were collected and combined. After removing the solvent under reduced pressure, 2.4 mg purified hassallidin A were obtained.

Hassallidin A (1): white, amorphous solid; UV (MeOH) $\lambda_{max}$ 226 nm (ε 5500), 278 nm (ε 880), 265 nm (ε 700); IR $\nu_{max}$ (film): 3448, 3343, 3282, 3080, 2927, 2853, 1740, 1658, 1617, 1543, 1530, 1527, 1453, 1384, 1268, 1240, 1232, 1172, 1128, 1091, 1067 $cm^{-1}$; $^1H$ and $^{13}C$ NMR, see Table 1; HR-ESI-FTICR-MS m/z $[M+Na]^+$ 1404.67572 (calcd for $[C_{62}H_{99}N_{11}O_{24}Na]^+$ 1404.67618, relative mass error $\Delta_m$= 0.33 ppm).

MS Analysis. Mass spectral analyses were performed on a matrix-assisted laser desorption/ionisation time-of-flight (MALDI-TOF) and a liquid chromatography-electrospray-ionisation (LC-ESI) mass spectrometer.

Sample application for MALDI-TOF measurements was carried out directly on sample plates with a mixture of 1 μL matrix (saturated 2,5-dihydroxybenzoic acid in 50% acetonitrile, 0.3% TFA) and 1 μL of a 50% MeOH solution containing about 0.2 μg hassallidin A.

The monoisotopic mass of hassallidin A was determined in positive ion reflector mode by using delayed extraction (DE). To obtain additional structural information the fragmentation pattern w as recorded using the post-source decay (PSD) modus a nd stepwise lowering the reflector voltage. Below m/z 300 air was introduced into the collision cell, which increased the number of small fragments and immonium ions. The liquid chromatography system of the LC-ESI-MS were performed with an [Agilent Zorbax SB-$C_{18}$, 5 μm, 4.6 x 150 mm column; mobile phase: solvent A: $H_2O$/TFA (0.02%), solvent B: acetonitrile/TFA (0.02%), 0.5 mL/min; UV detection at 220 nm]. The gradient was: solvent B from 20% to 100% in 20 min and isocratic 5 min. The desired mass signal appeared at 11.5 -13.0 min. The injection volume was 4 μL (about 80 pmol hassallidin A) of a 50% MeOH solution. The MS spectra were generated on a dual octopole ion trap mass spectrometer operated in positive ion mode and fitted with an atmospheric pressure electrospray-ionisation sample introduction device. Fragmentation experiments were performed by automatic $MS^{(n)}$ technique.

Chiral Amino Acid Analysis. Approx. 50 nM of sample was hydrolysed in 200 μL 6 N HCl (110 °C/24 h). The dry hydrolysate was derivatized to the *N*-(*O*-) trifluoroacetyl/ethyl ester and analysed by GC-MS (Agilent 6890/5973 MSD) using a 20 m x 0.25 mm Lipodex E / PS255 (30:70) capillary column. Quantitative analysis was performed by enantiomer labeling.[24]

Sugar and Fatty Acid Analysis. The sample was heated at 70°C for 16 h with 0 .65 N HCl/abs. M eOH. Excess m ethanol w as evaporated off. The dry residue was treated with *N,O*-bis(trimethylsilyl)- trifluoroacetamide (BSTFA)/ acetonitrile (1:1) (60 °C/30 min) and the derivatized sugar and fatty acid were analysed directly by GC-MS on a DB-5 capillary (J + W, Folson).

**References and Notes**

(1) Blom, J. F.; Bister, B.; Bischoff, D.; Nicholson, G.; Jung, G.; Süssmuth, R. D.; Jüttner, F. *J. Nat. Prod.* **2003,** *66,* 431-434.

(2) Burja, A. M.; Banaigs, B.; Abou-Mansour, E.; Burgess, J. G.; Wright, P. C. *Tetrahedron* **2001,** *57,* 9347-9377.

(3) Lesser, M. P.; Mazel, C. H.; Gorbunov, M. Y.; Falkowski, P. G. *Science* 2004, 13; 305(5686): 997-1000.

(4) Proksch, P.; Edrada, R. A.; Ebel, R. *Appl. Microb. Biotechnol.* **2002,** 59, 125-134.

(5) Namikoshi, M.; Rinehart, K. L. J. *Ind. Microb. Biotechnol.* **1996**,17,373-384.

(6) Guyot, M.; Dore, J. C.; Devillers, J. *SAR and QSAR in Environmental Research* **2004,** *15(2),*101-114.

(7) De Lucca, A. J.; Walsh, T. J. *Antimicrob. Agents Chemother.* ***1999,*** *43,* 1-11.

(8) Geitler, L. In *Synoptische Darstellung der Cyanophyceen in morphologischer und systematischer Hinsicht;* Beih. Bot. Centralbl. 1932; Chapter 2, pp 163-324.

(9) Welker, M.; Christiansen, G.; von Döhren, H. *Arch. Microbiol.* **2004,** 182, 288-298.

(10) Harvey, D. J. *Mass Spectrom. Rev.* **1999,** 18, 349-451.

(11) Takayama, M. *J. Am. Soc. Mass Spectrom.* ***2001,*** 12(4): 420-7.

(12) Piantini, U.; Sorensen, O. W.; Ernst, R. R. *J. Am. Chem. Soc.* **1982,** *104,* 6800-6801.
(13) Jeener, J.; Meier, B. H.; Bachmann, P.; Ernst, R. R. *J. Chem. Phys.* **1979,** *71,* 4546-4553.
(14) Braunschweiler, L.; Ernst, R. R. *J. Magn. Reson.* **1983,** *53,* 521-528.
(15) Bax, A.; Davis, D. G. *J. Magn. Reson.* **1985,** *65*, 355-360.
(16) Bax, A.; Griffey, R. H.; Hawkins, B. L. *J. Am. Chem. Soc.* **1983,** *105,* 7188-7190.
(17) Kessler, H.; Schmieder, P.; Kurz, M. *J. Magn. Reson.* **1989,** *85,* 400-405.
(18) Lerner, L.; Bax, A. *J. Magn. Reson.* **1986,** *69,* 375-380.
(19) Clore, G. M.; Bax, A.; Wingfield, P.; Gronenborn, A. M. *Febs Letters* **1988,** *238,* 17-21.
(20) Kessler, H.; Schmieder, P.; Kock, M.; Reggelin, M. *J. Magn. Reson.* **1991,** *91,* 375-379.
(21) Bax, A.; Subramanian, *S. J. Magn. Reson.* **1986,** *67, 565-569.*
(22) Bax, A.; Summers, M. F. *J. Am. Chem. Soc.* **1986,** *108,* 2093-2094.
(23) Bodenhausen, G.; Ruben, D. J. *Chem. Phys. Lett.* **1980**, *69,* 185-189.
(24) Frank, H.; Nicholson, G. J.; Bayer, E. *J. Chromatogr.* **1978**, *167,* 187-196.
(25) National Committee for Clinical Laboratory Standards, Reference method for broth dilution antifungal susceptibility testing of filamentous fungi; approved standard M38-P, 1998; Vol. 22, Number 16.
(26) National Committee for Clinical Laboratory Standards, 1992, Reference method for broth dilution antifungal susceptibility testing for yeast; proposed standard M27-A2. National Committee for Clinical Laboratory Standards, Villanova, Pa.

The features of the present invention disclosed in the specification, the claims and/or in the accompanying drawings, may, both separately, and in any combination thereof, be material for realising the invention in various forms thereof.

## Claims

1. A compound of formula

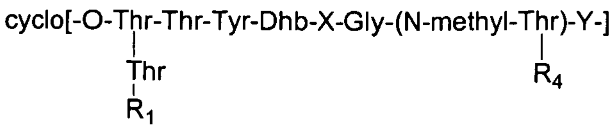

,

wherein
Dhb = 2-amino-2-dehydrobutyric acid
X = glutamine, glutamic acid, or ornithine
Y = glutamine, glutamic acid, or ornithine, or

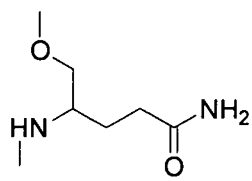

,

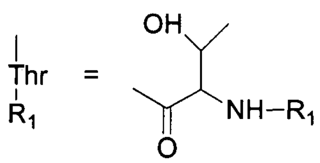

,

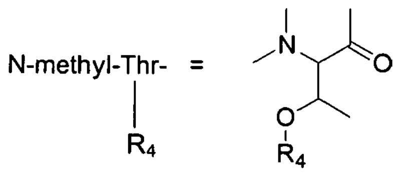

,

$R_1$ = 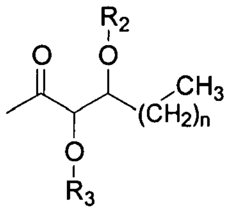

,

or H, wherein n = 0 - 12,
$R_2$ = hexose, hexulose, pentose, sialic acid or H, in particular rhamnose,
$R_3$= hexose, hexulose, pentose, sialic acid or H, in particular rhamnose,
$R_4$ = hexose, hexulose, pentose, sialic acid or H, in particular mannose.

**2.** The compound according to claim 1, wherein, independently, each amino acid is in its D- or L -form, or, in the case of Dhb, in its E- or Z-form.

**3.** The compound according to any of the foregoing claims, wherein one or several of the OH-groups and/or $NH_2$-groups may be one or several of the following: methylated, phosphorylated, glycosylated, sulfonated, acetylated and, in the case of OH-groups, modified with a $-(CH_2)_2-NH_2$-residue, and/or wherein one or several of the NH-groups may be methylated.

**4.** The compound according to any of the foregoing claims wherein 2-amino-2-dehydrobutyric acid has been modified such that it has the formula:

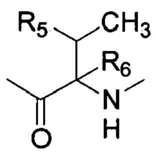

$R_5$=H,Cl,Br,OH
$R_6$ = H, Cl, Br, OH.

**5.** The compound according to any of the foregoing claims, wherein one of the threonine residues is D-threonine, one is L-threonine, and one is D-allo-threonine, wherein X, Y and N-methyl-Thr are in their D- or L-forms, and wherein Tyr is in its D-form.

**6.** The compound according to any of the foregoing claims, wherein $R_4$ is mannose, $R_1$ is

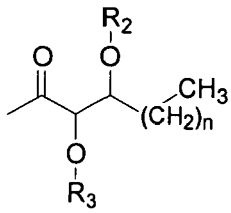

,

herein n = 10,
$R_2$ = rhamnose or H,
and $R_3$ = H.

**7.** The compound according to any of the foregoing claims, wherein X is Gin and Y is Gln.

**8.** A compound, preferably according to any of the foregoing claims, having the formula

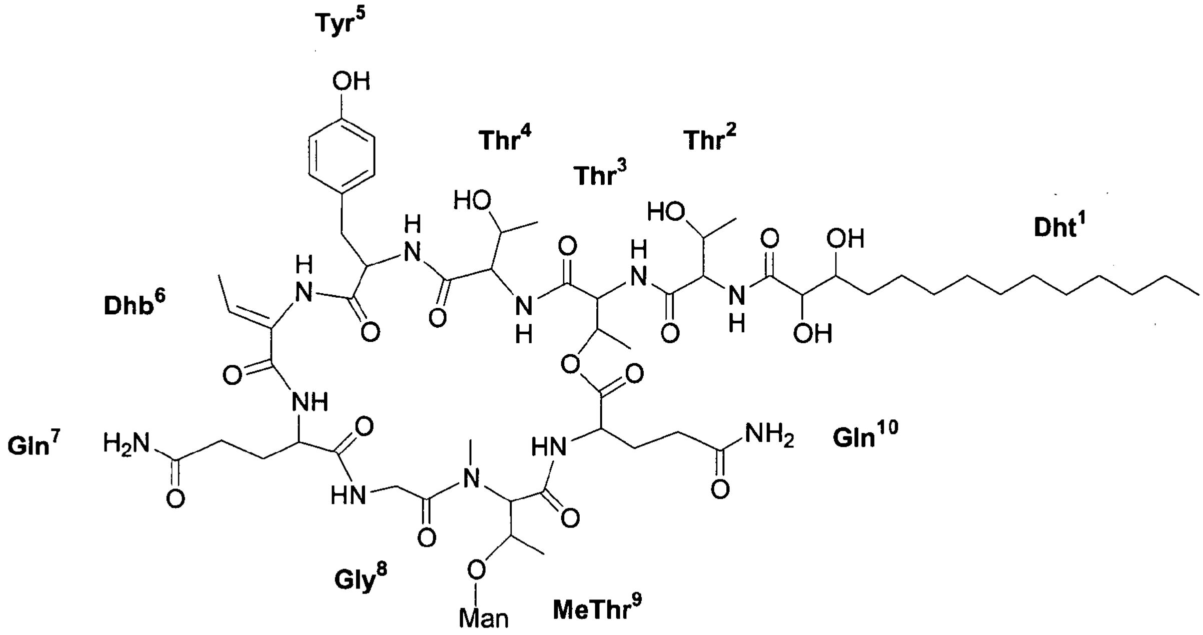

**9.** A compound, preferably according to any of the forgoing claims, having the formula

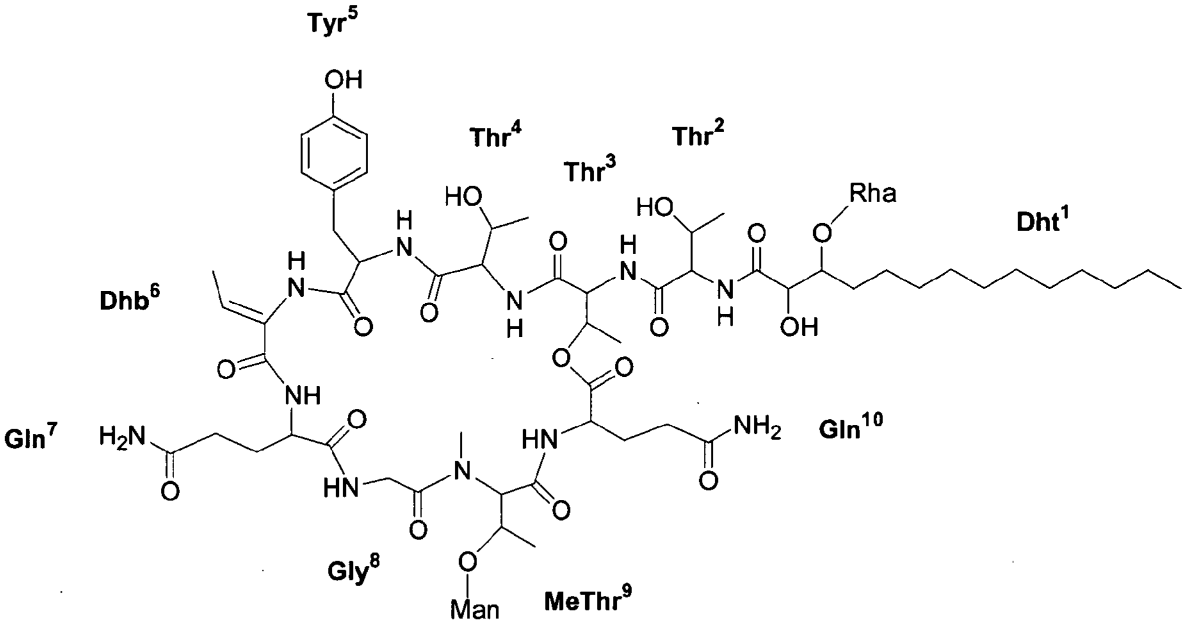

**10.** Pharmaceutical composition comprising a compound according to any of the foregoing claims.

**11.** Pharmaceutical composition according to claim 10, additionally comprising a pharmaceutically acceptable carrier.

**12.** Use of a compound according to any of claims 1 - 9 for the manufacture of a medicament for the treatment of a fungal disease.

**13.** Use of a compound according to any of claims 1 - 9 for the manufacture of a medicament for the treatment of a cancerous disease.

**14.** A method of isolating a compound according to any of claims 1 - 9, comprising the following steps:

- growing cyanobacterium HAS BO7, having the official DSMZ accession no. DSM 17156, or *Tolypothrix* sp. in culture,
- extracting the cultivated cyanobacteria with a solvent, preferably an alcoholic solvent, more preferably methanol or ethanol,
- subjecting the resulting extract to a reverse-phase chromatography on HPLC, more preferably eluting with an increasing amount of a non-polar solvent.

**15.** The method according to claim 14, further comprising the additional step:

- identifying the compound by UV-detection at 220 nm.

**16.** Cyanobacterium, as deposited with the DSMZ (Braunschweig, Germany, Deutsche Sammlung für Mikroorganismen und Zellkulturen), under applicant's reference sign HAS BO7, and having the official DSMZ accession no. DSM 17156.

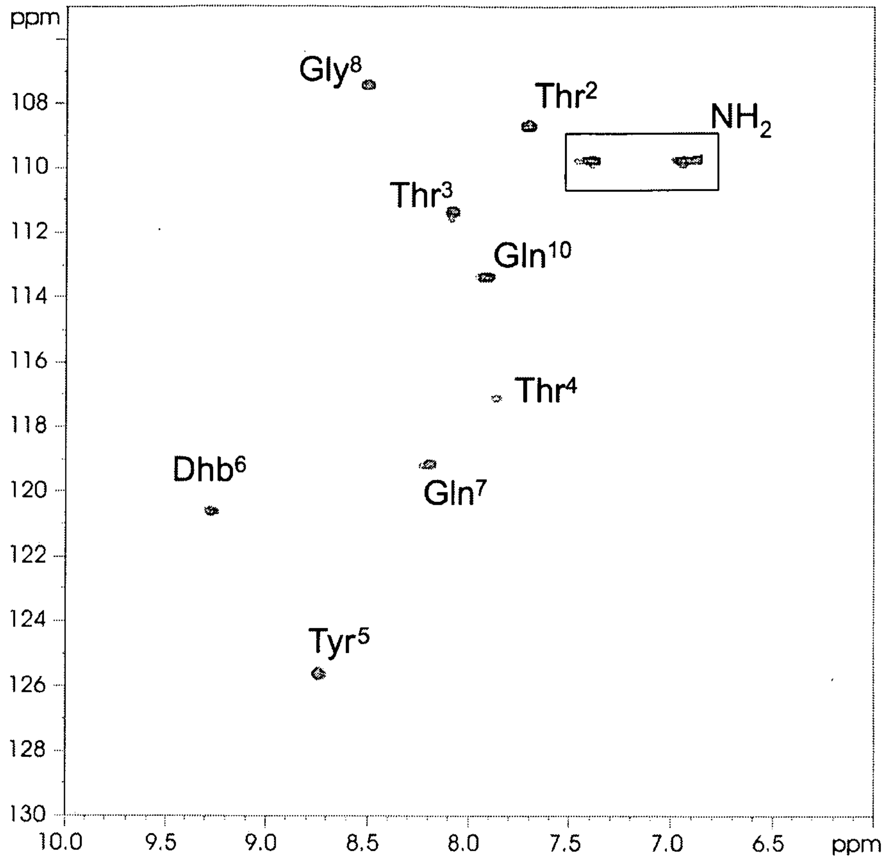
ppm
108
110
112
114
116
118
120
122
124
126
128
130
10.0
9.5
9.0
8.5
8.0
7.5
7.0
6.5
ppm
Gly8
Thr2
NH2
Thr3
Gln10
Thr4
Dhb6
Gln7
Tyr5

Fig. 1

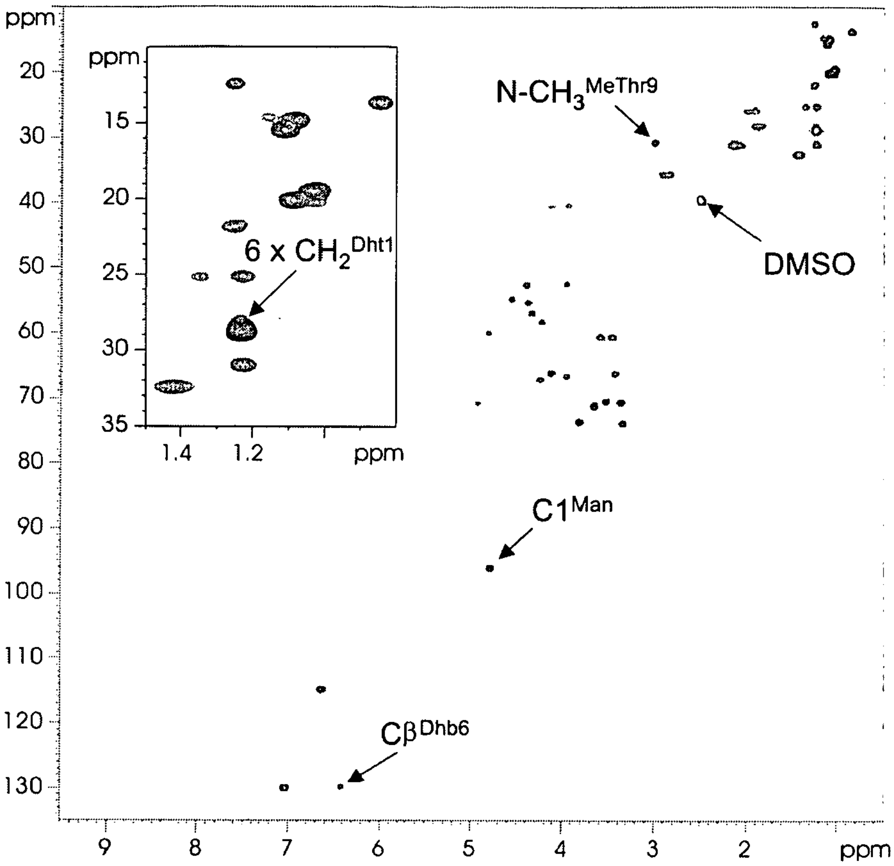


Fig. 2

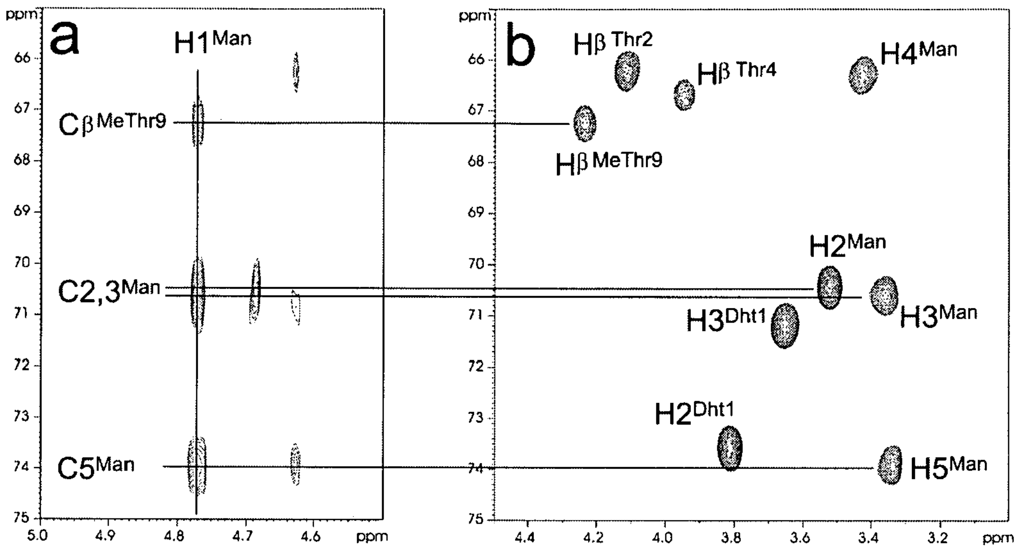
a
H1Man
CβMeThr9
C2,3Man
C5Man
b
Hβ Thr2
Hβ Thr4
H4Man
Hβ MeThr9
H2Man
H3Dht1
H3Man
H2Dht1
H5Man
ppm

Fig. 3

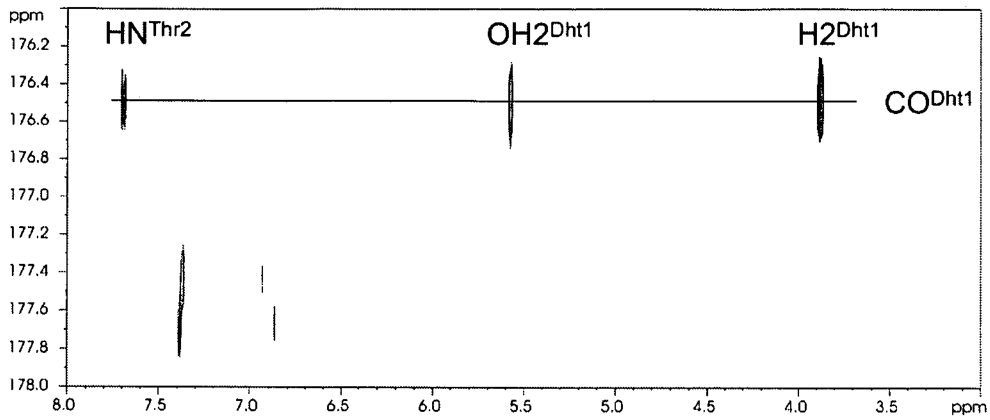
ppm
176.2
176.4
176.6
176.8
177.0
177.2
177.4
177.6
177.8
178.0
8.0
7.5
7.0
6.5
6.0
5.5
5.0
4.5
4.0
3.5
ppm
HNThr2
OH2Dht1
H2Dht1
CODht1

Fig. 4

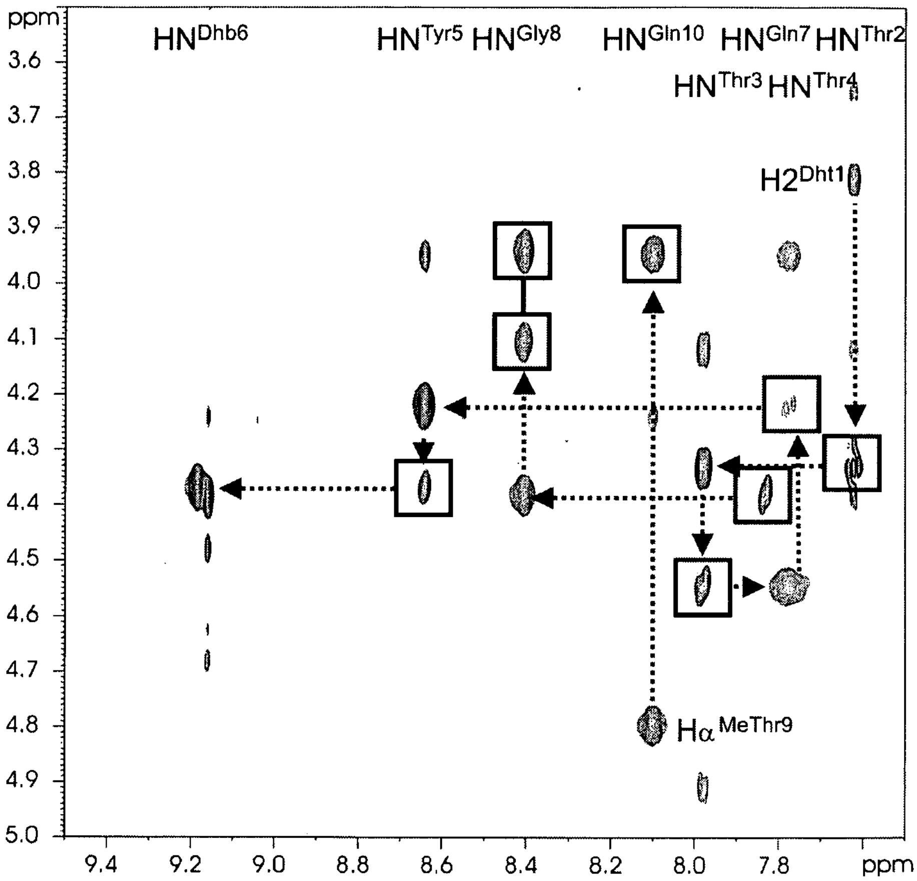
ppm
HN^Dhb6
HN^Tyr5 HN^Gly8
HN^Gln10 HN^Gln7 HN^Thr2
HN^Thr3 HN^Thr4
H2^Dht1
Hα^MeThr9
3.6
3.7
3.8
3.9
4.0
4.1
4.2
4.3
4.4
4.5
4.6
4.7
4.8
4.9
5.0
9.4
9.2
9.0
8.8
8.6
8.4
8.2
8.0
7.8
ppm

Fig. 5

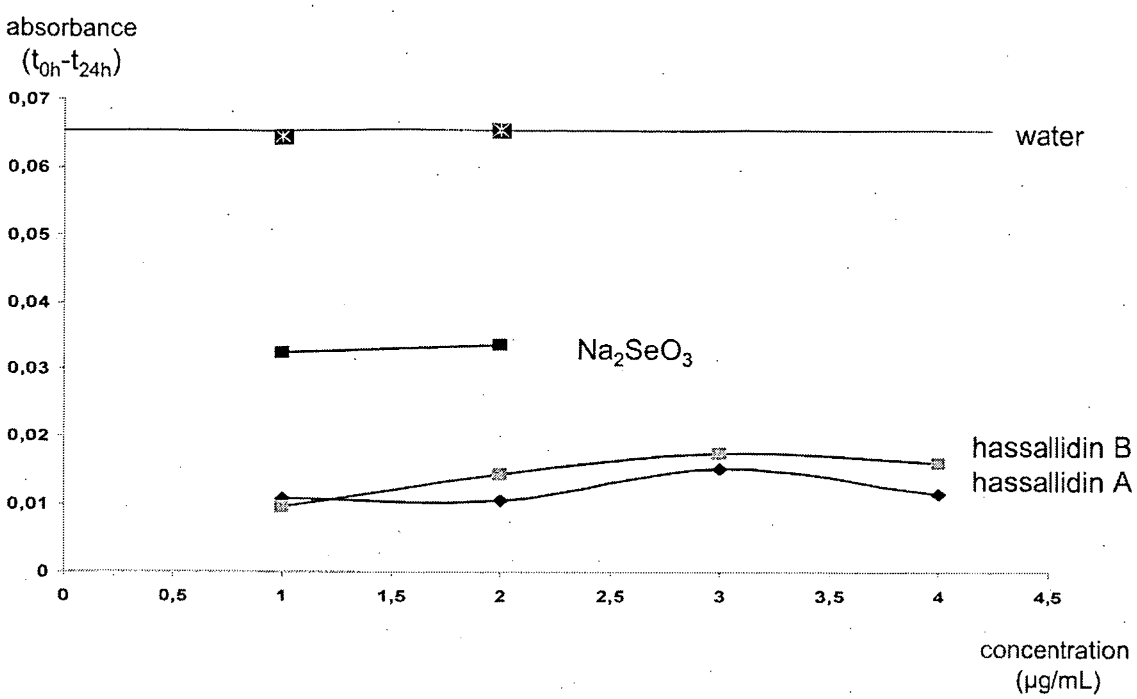
absorbance
($t_{0h}$-$t_{24h}$)
0,07
0,06
0,05
0,04
0,03
0,02
0,01
0
0
0,5
1
1,5
2
2,5
3
3,5
4
4,5
water
$Na_2SeO_3$
hassallidin B
hassallidin A
concentration
(µg/mL)

Fig.6

European Patent Office

# EUROPEAN SEARCH REPORT

Application Number
EP 05 00 4582

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | US 4 137 224 A (TAPLIN ET AL) 30 January 1979 (1979-01-30) Abstract; col. 3-col. 4 ----- | 1-16 | C07K9/00 A61K38/14 C12P21/00 C12R1/01 A61P31/00 A61P35/00 |
| A | BURJA A M ET AL: "Marine cyanobacteria-a prolific source of natural products" TETRAHEDRON, ELSEVIER SCIENCE PUBLISHERS, AMSTERDAM, NL, vol. 57, no. 46, 12 November 2001 (2001-11-12), pages 9347-9377, XP004312068 ISSN: 0040-4020 * the whole document * ----- | 1-16 | |
| A | PAPENDORF O ET AL: "Hierridin B and 2,4-dimethoxy-6-heptadecyl-phenol, secondary metabolites from the cyanobacterium Phormidium ectocarpi with antiplasmodial activity" PHYTOCHEMISTRY, PERGAMON PRESS, GB, vol. 49, no. 8, 20 December 1998 (1998-12-20), pages 2383-2386, XP004290443 ISSN: 0031-9422 p. 2385, col. 1, "Extraction and isolation". ----- | 14 | TECHNICAL FIELDS SEARCHED (Int.Cl.7) C07K |
| X,P | NEUHOF TORSTEN ET AL: "Hassallidin A, a glycosylated lipopeptide with antifungal activity from the cyanobacterium Hassallia sp." JOURNAL OF NATURAL PRODUCTS. MAY 2005, vol. 68, no. 5, May 2005 (2005-05), pages 695-700, XP002334646 ISSN: 0163-3864 * the whole document * ----- | 1-16 | |

The present search report has been drawn up for all claims

2

| Place of search | Date of completion of the search | Examiner |
|---|---|---|
| Munich | 5 July 2005 | Lopez García, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 05 00 4582

This annex lists the patent family members relating to the patent documents cited in the above-mentioned European search report.
The members are as contained in the European Patent Office EDP file on
The European Patent Office is in no way liable for these particulars which are merely given for the purpose of information.

05-07-2005

| Patent document cited in search report | Publication date | Patent family member(s) | Publication date |
|---|---|---|---|
| US 4137224 A | 30-01-1979 | US 4230799 A | 28-10-1980 |
| | | US 4232006 A | 04-11-1980 |

EPO FORM P0459

For more details about this annex : see Official Journal of the European Patent Office, No. 12/82